# EUROPÄISCHE PATENTSCHRIFT

(11) **EP 0 675 186 B1**
(45) Veröffentlichungstag und Bekanntmachung des Hinweises auf die Patenterteilung: **20.01.1999**
(21) Anmeldenummer: 95104007.0
(22) Anmeldetag: 18.03.1995
(51) Int. Cl.: C09K 19/20, C09K 19/22, C09K 19/38, G02F 1/1335, C07C 235/50, C07C 251/24, C07C 69/92

(54) **Photovernetzbare Flüssigkristalle**
Photo-curable liquid crystals
Cristaux liquides photo-réticulables

(30) Priorität: 30.03.1994 CH 953/94
(43) Veröffentlichungstag der Anmeldung: 04.10.1995
(73) Patentinhaber: Rolic AG, 6301 Zug (CH)
(72) Erfinder: Kelly, Stephen, CH-4313 Möhlin (CH); Schadt, Martin, CH-4410 Seltisberg (CH); Schmitt, Klaus, D-79541 Lörrach (DE)
(74) Vertreter: England, Christopher David

(56) Entgegenhaltungen:
- EP-A- 0 501 563
- LIQUID CRYSTALS, Bd. 14, Nr. 1, 1993 LONDON GB, Seiten 15-36, A.SIRIGU 'plenary lecture: topics in the evolution of liquid crystal polymers'

## Beschreibung

Die vorliegende Erfindung betrifft photovernetzbare Flüssigkristalle, flüssigkristalline Gemische, die solche Verbindungen enthalten, sowie ihre Verwendung in vernetztem Zustand für optische Bauelemente.

Photovernetzbare Flüssigkristalle versehen mit einer geeigneten Menge eines Photinitiators können auf einem Substrat wie Glas oder Silizium von einer Orientierungsschicht oder in einem elektrischen oder magnetischen Feld orientiert und dann durch Bestrahlen mit Licht einer geeigneten Wellenlänge vernetzt werden. Die dadurch erzeugte Orientierung bleibt auch bei hohen Temperaturen erhalten. Dies gilt auch, wenn die Orientierungsschicht aus photoorientierbaren Polymernetzwerkschichten (PPN) besteht, welche durch selektive Bestrahlung mit polarisiertem UV-Licht hochaufgelöste Orientierungsmuster auf flüssigkristallinen Schichten induzieren. Solche Hybridschichten ermöglichen es, die optisch eingeschriebene hochaufgelöste Orientierungsstruktur einer dünnen Polymerschicht auf eine doppelbrechende dichtvernetze Polymerschicht beliebiger Dicke zu übertragen. Mischungen bestehend aus photovernetzbaren Flüssigkristallen oder photovernetzbaren Flüssigkristallen und niedermolekularen Flüssigkristallen und/oder optisch aktiven (chiralen) Zusätzen werden je nach Anwendung verwendet. So lassen sich langzeitstabile optische Bauelemente, wie zum Beispiel optische Retarder, Wellenleiter, optische Gitter und Filter, integrierte Farbfilter, piezoelektrische optische Bauelemente und solche mit nicht-linearen optischen (NLO) Eigenschaften, usw. herstellen. Solche optischen Bauelemente finden zum Beispiel in Projektionssystemen Einsatz.

In der Publikation "Topics in the evolution of liquid crystal polymers" von A. Sirigu (Liquid Crystals, 1993, Vol. 14, No. 1, 15-36) sind photovernetzbare Flüssigkristalle offenbart, welche für die vorstehend genannten Zwecke verwendbar sind.

Weitere Eigenschaften, wie beispielsweise die Doppelbrechung, die Brechungsindices, die Transparenz, usw. müssen je nach Anwendungsgebiet unterschiedlichen Anforderungen genügen. Beispielsweise sollten Materalien für optische Retarder eine hohe Doppelbrechung aufweisen, damit die Schichtdicke der integrierten optischen Bauelemente auf einem Minimum gehalten werden kann.

Neben dem generellen Interesse an photovernetzbaren Flüssigkristallen für optische Bauelemente, eignen sich solche flüssigkristallinen Materialien als Cladding von Glasfibern für optische Datenübertragung, so wie das zum Beispiel aus der EP-A 0 501 563 bekannt ist. Der Einsatz solcher Materialien erhöht den elastischen Modulus in der Längsachse des Fibers, verkleinert die thermischen Expansionskoeffizienten und vermindert Mikroverbiegungsverluste. Dies führt zu einer erhöhten mechanischen Stabilität.

Die photovernetzbaren Flüssigkristalle müssen eine gute chemische und thermische Stabilität, gute Löslichkeit in gängigen Lösungsmitteln und eine gute Stabilität gegenüber elektrischen Feldern und elektromagnetischer Strahlung besitzen. Weiterhin sollten sie von etwa 25°C bis etwa 80°C, wenn möglich von etwa 25°C bis etwa 100°C eine geeignete Mesophase besitzen, beispielsweise für die oben genannten Anwendungen eine breite smektische oder nematische Mesophase, bzw. chiral smektische und cholesterische Mesophase.

Da Flüssigkristalle in der Regel als Mischungen mehrerer Komponenten zur Anwendung gelangen, ist es wichtig, dass die Komponenten untereinander gut mischbar sind.

Die vorliegende Erfindung stellt nun Verbindungen zur Verfügung, die in hervorragender Weise als Einzelkomponenten oder als Komponenten von Flüssigkristall-Mischungen für solche optischen Bauelemente geeignet sind. Gegenstand der vorliegenden Erfindung sind somit Verbindungen der allgemeinen Formel 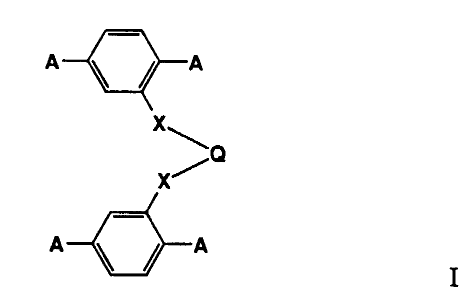 worin
- X: eine Einfachbindung, -COO-, -CH₂O-, -CONH- oder -C=N-;
- Q: n-Alkylen, c-Alkylen, 1,4-Phenylen, 4,4'-Biphenylen oder 2,6-Naphthylen bedeuten; und

Der mesogene Reste A bedeutet 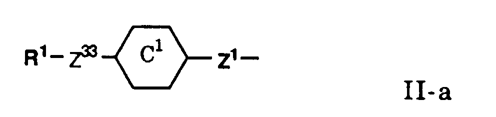 worin
- R¹: Acrylat, Methacrylat, 2-Chloracrylat, 2-Phenylacrylat, Vinyloxy oder Epoxy bedeutet;
- Z³³: -(CH₂)_{m'}-, -(CH₂)_{m'}O-, -(CH₂)_{m'}COO- oder -(CH₂)_{m'}OOC- bedeutet;
- m': eine ganze Zahl von 4 bis 12 ist;
- Ring C¹: ein gegebenenfalls mit Halogen substituiertes 1,4-Phenylen, oder trans-1,4-Cyclohexylen bedeutet; und
- Z¹: -CH₂CH₂-, -CH₂O-, -COO-, -OOC-, -(CH₂)₄- oder -(CH)₃O-

Die Verbindungen der allgemeinen Formel I zeichnen sich durch ihre verhältnismässig niedrige Viskosität aus. Sie können daher problemlos auf eine geeignete Oberfläche aufgetragen werden. Dies geschieht in der Regel durch Spin-Coating. Da die erfindungsgemässen Verbindungen zudem eine flüssigkristalline Phase aufweisen, können sie vor dem Vernetzen durch Anlegen eines elektrischen Feldes ausgerichtet werden.

Ganz besonders bevorzugt sind die mesogenen Reste, worin Ringe C und D unabhängig voneinander unsubstituiertes oder mit Fluor substituiertes 1,4-Phenylen, Pyridin-2,5-diyl, Pyrimidin-2,5-diyl oder trans-1,4-Cyclohexylen bedeuten; Z¹ -CH₂O-, -COO- oder -OOC- darstellt; und Z² eine Einfachbindung, -CH₂CH₂₋, -CH₂O-, -OCH₂₋, -COO- oder -OOC- bedeutet.

Der Ausdruck "n-Alkylen" bedeutet im Rahmen der vorliegenden Erfindung ein geradkettiges Alkylen mit 1 bis 16 Kohlenstoffatomen. Besonders bevorzugte Alkylene sind 1,4-Butylen, 1,5-Pentylen, 1,6-Hexylen, 1,7-Heptylen, 1,8-Octylen, 1,9-Nonylen, 1,10-Decylen, 1,11-Undecylen, 1,12-Dodecylen. Der Ausdruck "c-Alkylen" steht für ein cyclisches Alkylen mit 4 bis 8 Kohlenstoffatomen, wie beispielsweise 1,3-Cyclobutylen, 1,2- oder 1,3-Cyclopentylen, 1,4-Cyclohexylen, 1,4-Bicylo(2,2,2)octan und dergleichen.

"Gegebenenfalls mit Halogen, Methyl und/oder Cyano substituiertes 1,4-Phenylen" umfasst in der vorliegenden Erfindung 1,4-Phenylen, mit Fluor, Brom, Chlor, Methyl oder Cyano substituiertes 1,4-Phenylen wie beispielsweise 2- bzw. 3-Fluor-1,4-phenylen, 1,3-Difluor 3-Fluor-1,4-phenylen, 2,6-bzw. 3,5-Difluor-1,4-phenylen, 2- bzw. 3-Chlor-1,4-phenylen,2,3-Dichlor-1,4-phenylen, 2,6- bzw. 3,5-Dichlor-1,4-phenylen, 2- bzw. 3-Brom-1,4-phenylen, 2-bzw. 3-Methyl-1,4-phenylen, 2- bzw. 3-Cyano-1,4-phenylen und dergleichen.

Bevorzugte Reste R sind Acrylat, Methacrylat, 2-Chloracrylat, 2-Phenylacrylat, Acryloylphenylen, Acrylamid, Methacrylamid, 2-Phenylacrylamid, Vinylether, Vinylester, Styrol-Derivate, Maleinsäure-Derivate, Fumarsäure-Derivate und dergleichen. Es sind dies Reste, welche nach Beschichten des geeigneten Trägers mit Verbindungen der Formel I photochemisch vernetzt werden können.

Besonders bevorzugte Reste R sind Acrylat, Methacrylat, Acryloyloxy, Methacryloyloxy, Vinyloxy und Epoxy.

Vorzugsweise bedeutet X in Verbindungen der Formel I eine -COO-, -CONH- oder -CH₂O-Gruppe. Diese Gruppen sind aus dem entsprechenden Aldehyd leicht zugänglich und können dann ohne weiteres mit einem geeigneten Derivat der Gruppe Q, welche vorzugsweise -(CH₂)ₚ-, worin p eine ganze Zahl zwischen 2 und 12 ist, oder 1,4-Phenylen bedeutet, umgesetzt werden.

Der Mesophasen-Typ der erfindungsgemässen Verbindungen kann durch Variation der Ringe in den mesogenen Seitenketten A beeinflusst werden. So haben heterocyclische Ringe die Tendenz smektische Phasen zu erzeugen, während Cyclohexylen Ringe nematische Tendenzen fördern.

Ganz besonders bevorzugte Verbindungen der Formel I sind Verbindungen der Formeln 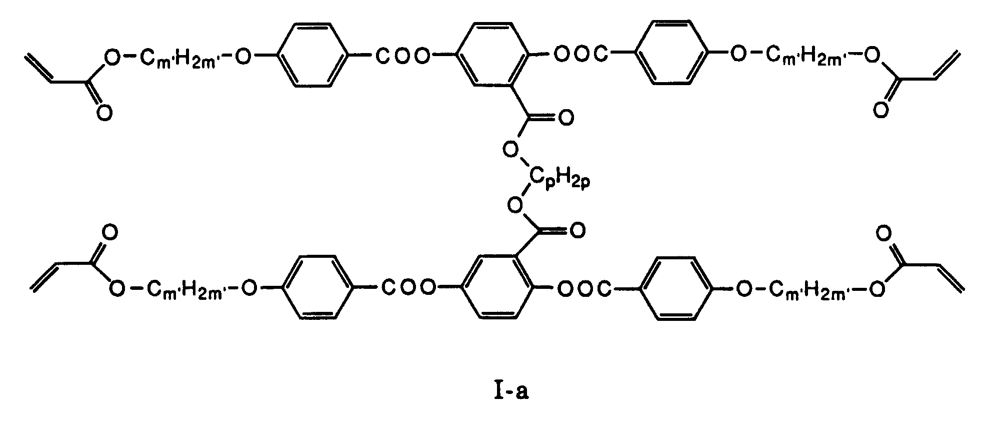 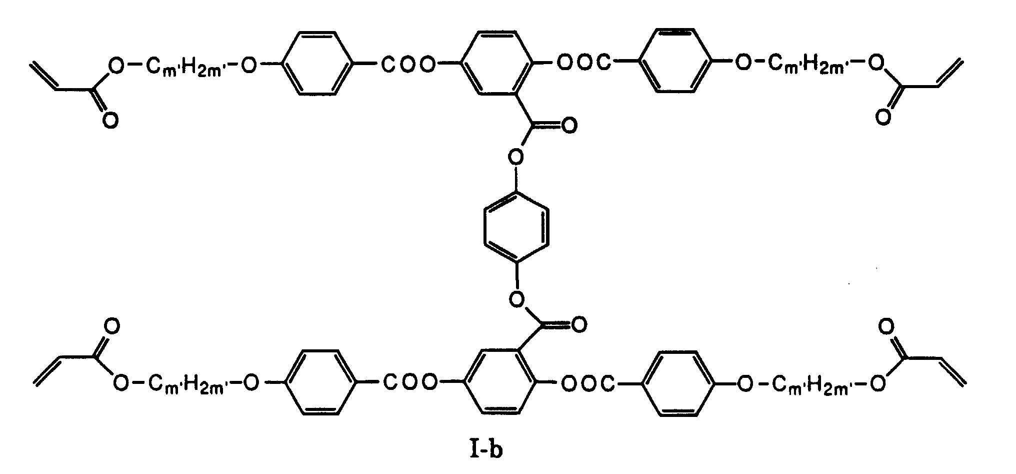 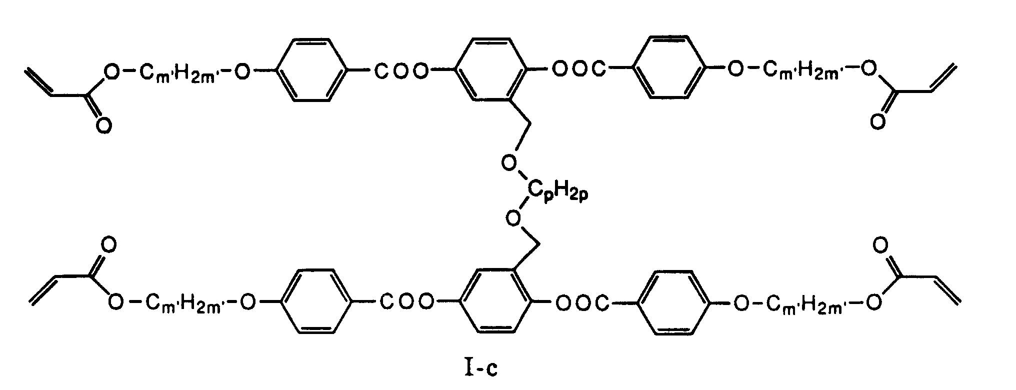 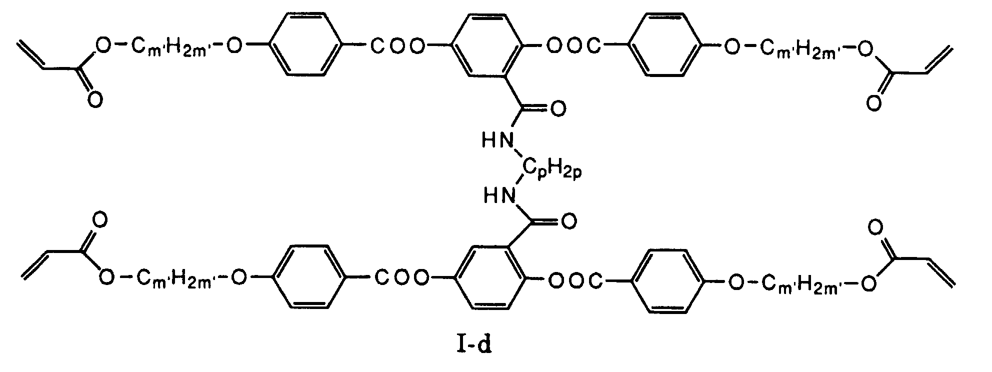 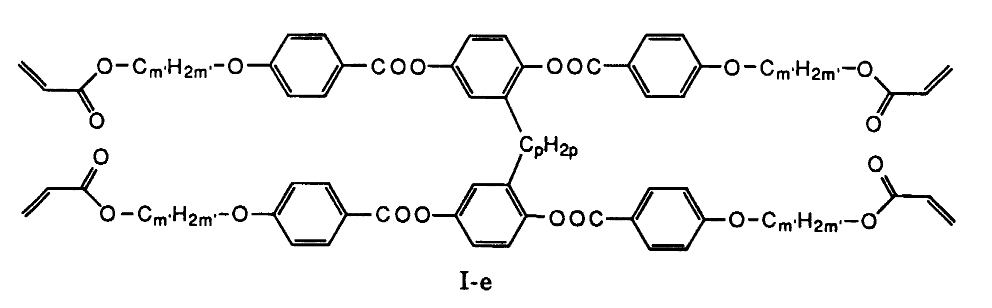 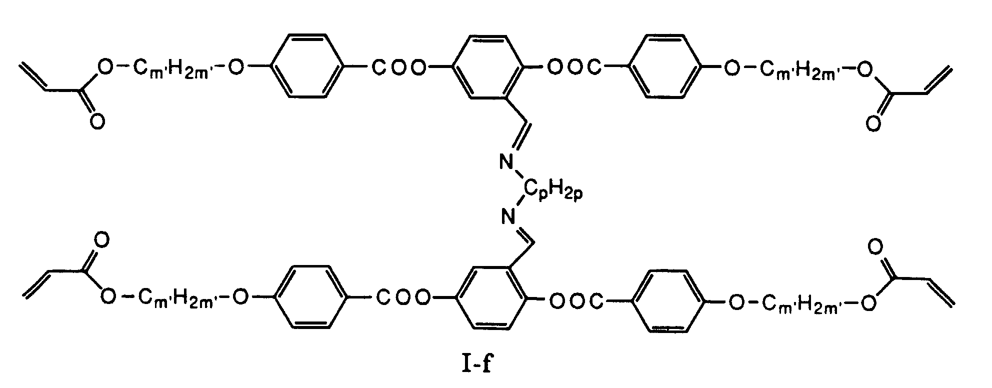 worin m' eine ganze Zahl von 4 bis 12 und p eine ganze Zahl von 2 bis 12 bedeutet.

Der Zugang zu den erfindungsgemässen Verbindungen ist ausserordentlich einfach. Sie können wie in den Schemata 1-6 aufgezeichnet ist, in an sich bekannter Weise hergestellt werden.

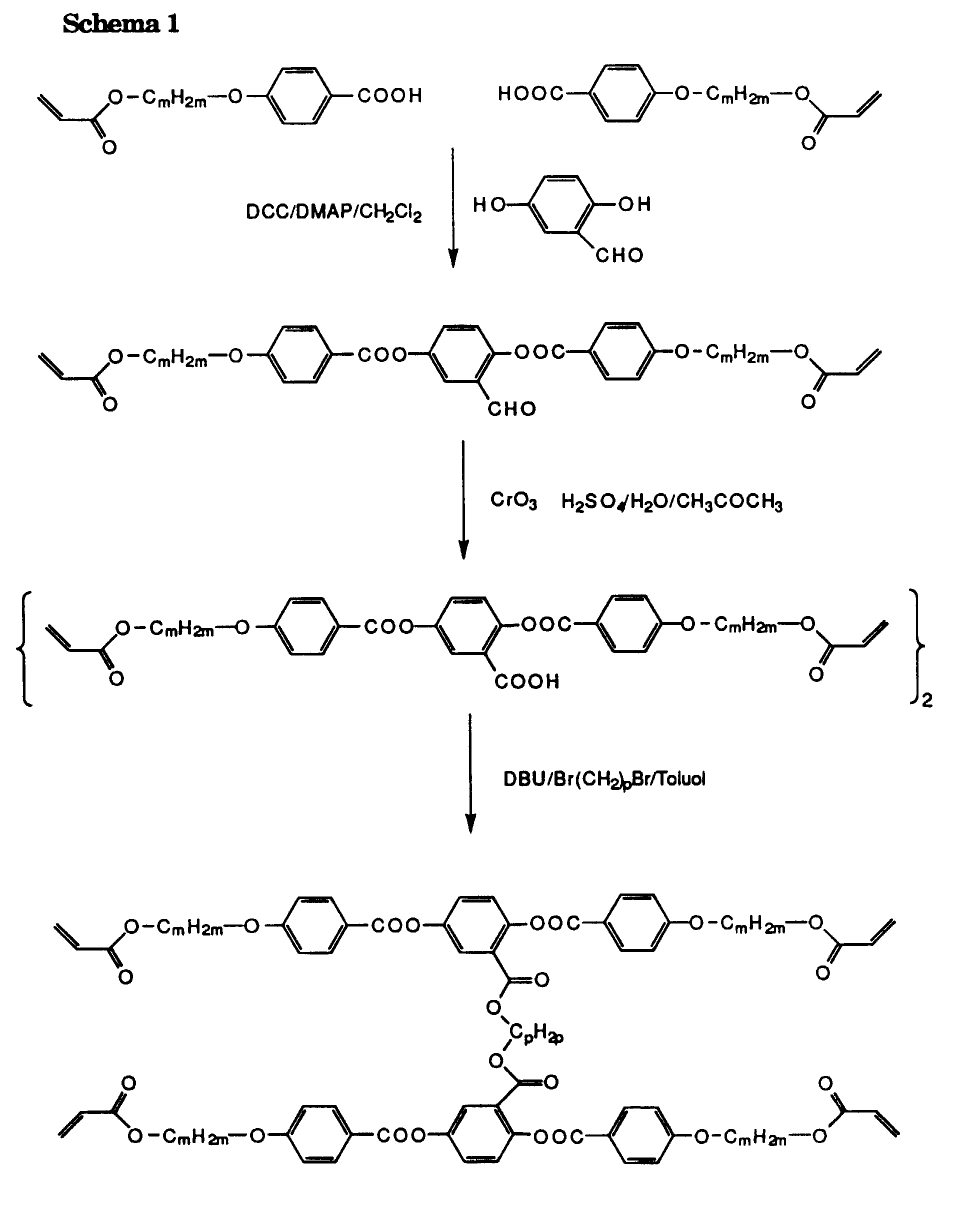

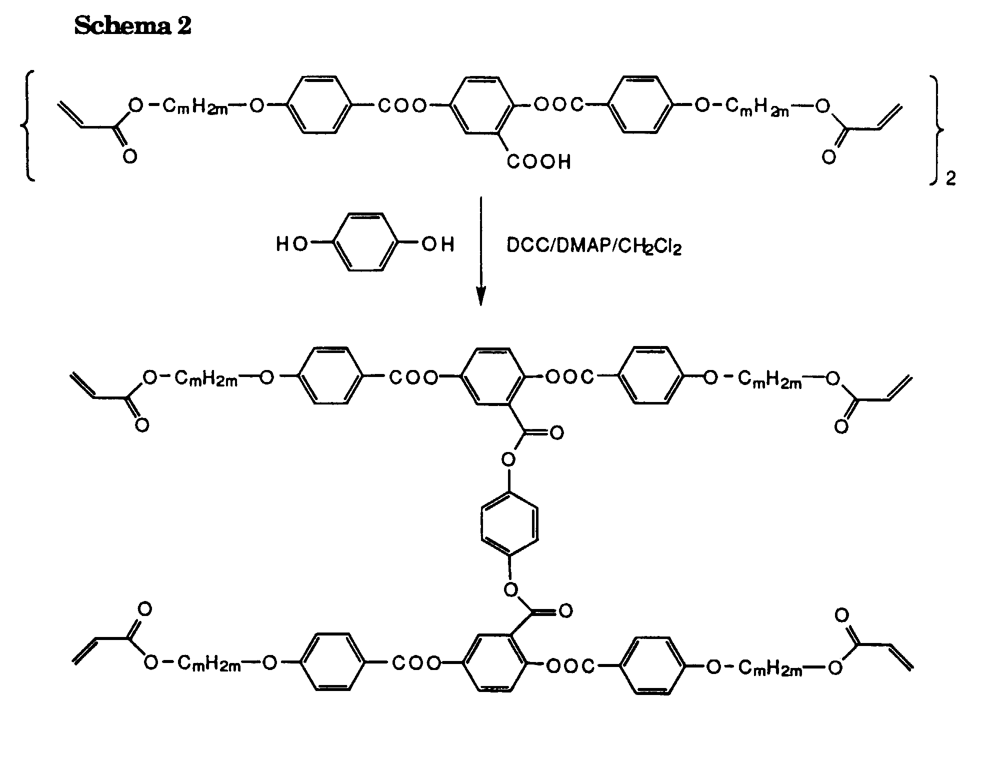

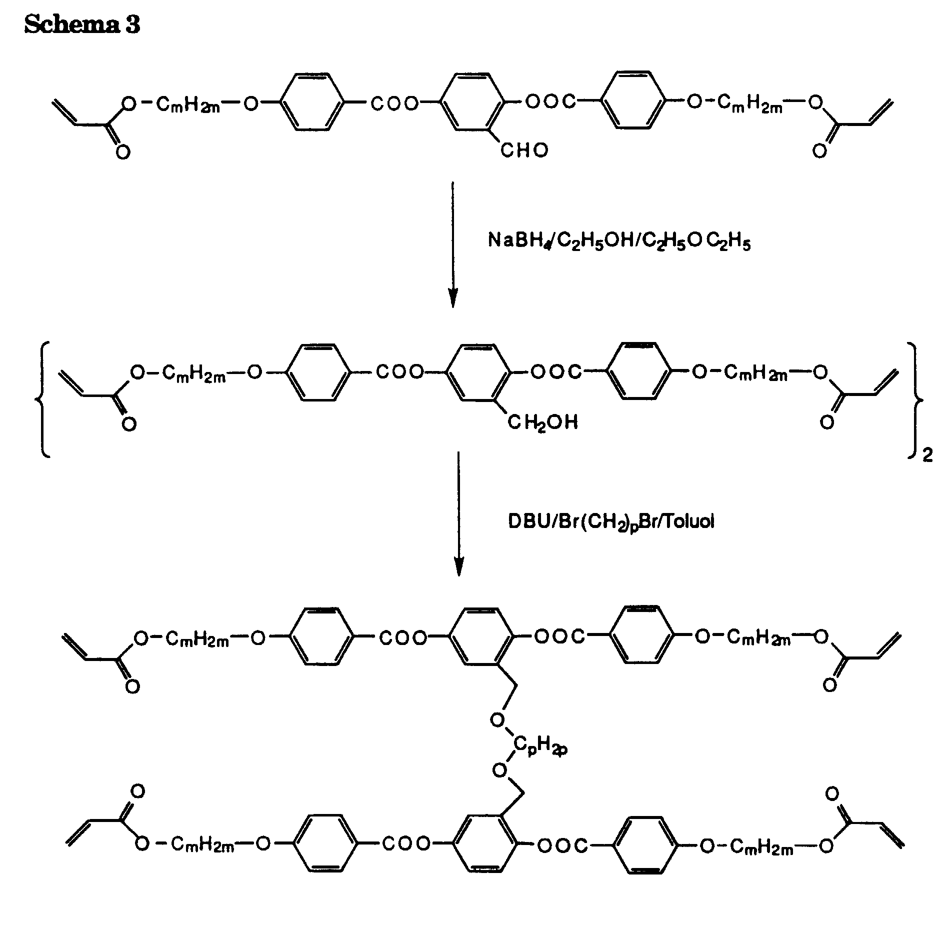

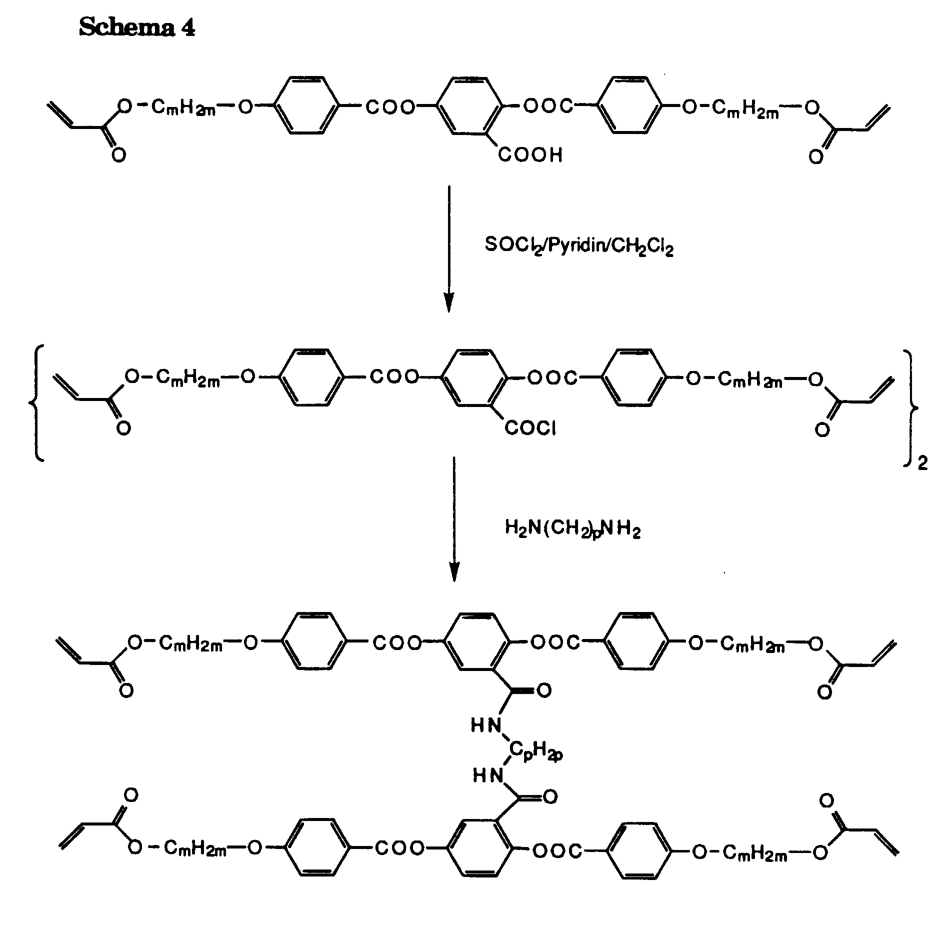

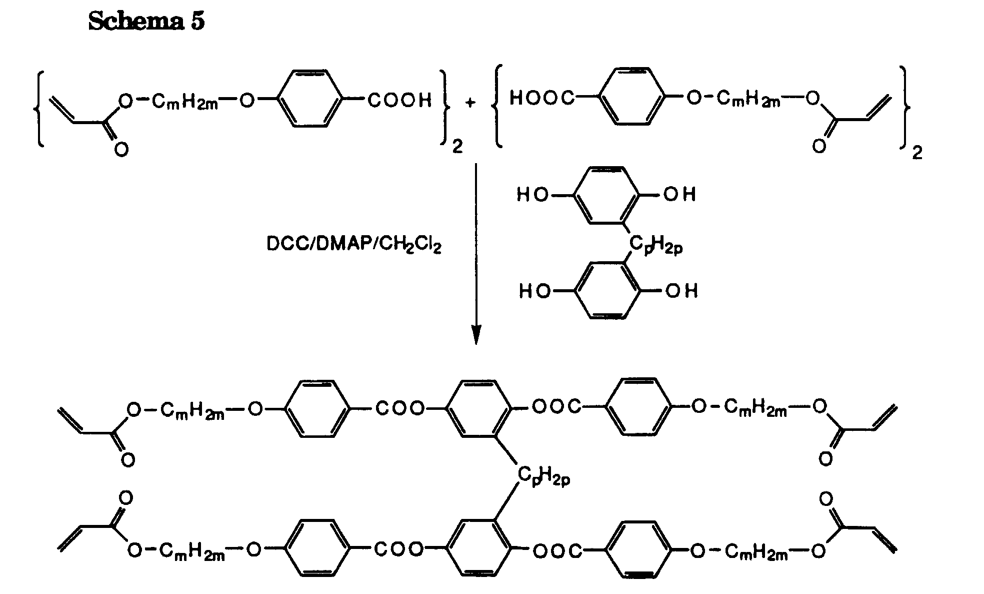

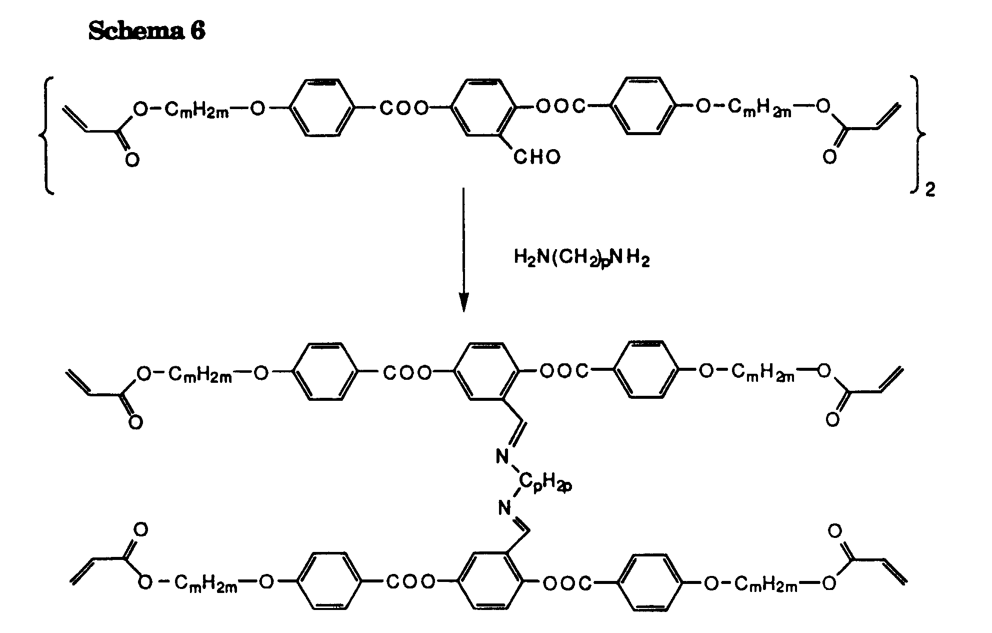

In den Schemata 1 bis 6 bedeutet m eine ganze Zahl von 1 bis 16 und p eine ganze Zahl von 2 bis 12.

Eine kleine Menge BHT (2, 6-Di-tert.-butyl-4-methyl-phenol/"Butylhydroxytoluol") wird jeder Stufe beigemischt, um unerwünschtes thermisches Vernetzen zu unterbinden.

Die Verbindungen der Formeln I können als reine Verbindungen, oder in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere, bekannte flüssigkristalline Verbindungen mit oder ohne photovernetzbare Gruppen sein. Es können auch eine oder mehrere chirale Komponenten im Gemisch enthalten sein.

Aufgrund der guten Löslichkeit der Verbindungen der Formel I und aufgrund ihrer guten Mischbarkeit untereinander kann der Anteil an verschiedenen Verbindungen der Formel I in den erfindungsgemässen Gemischen hoch sein und bis 100 Gew.-% betragen.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 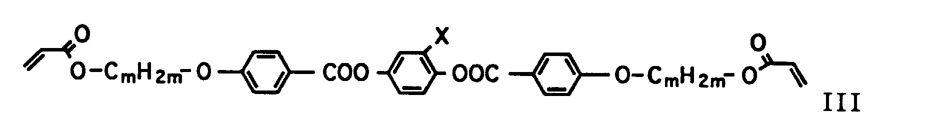 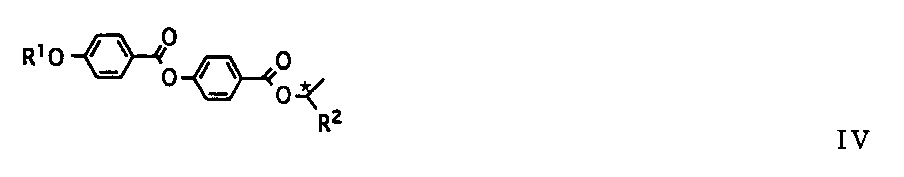 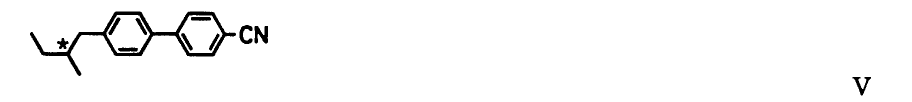 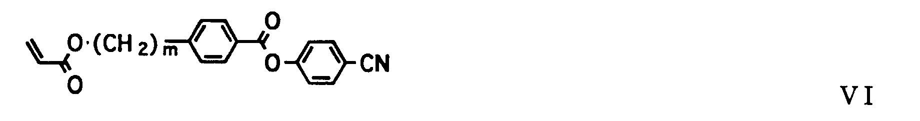 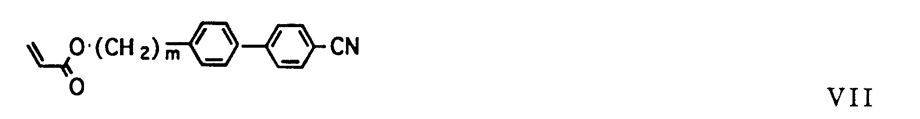 worin R¹ und R² unabhängig voneinander Alkyl oder Alkenyl mit 2 bis 12 Kohlenstoffatomen darstellen; X Wasserstoff, Niederalkyl, Fluor, Brom, Chlor oder Cyano darstellt; und m eine ganze Zahl von 1 bis 16 bedeutet.

Die Erfindung wird durch die folgenden Beispiele weiter veranschaulicht. In den Beispielen bedeutet C eine kristalline, N eine nematische, S eine smektische und I die isotrope Phase.

### Beispiel 1

Zu einer Lösung von 0,7 g 2,5-bis(4-[6-Acryloyloxyhexyloxy)]phenylcarbonyloxy)benzoesäure und 0,11 g 1,4-Dibrombutan in 20 ml Toluol wurde unter Rühren bei Raumtemperatur 0,15 g DBU (1,8-Diazobicyclo[5,4,0]-undec-1-en(1,5-5) gegeben. Das Reaktionsgemisch wurde über Nacht bei 80°C erwärmt, auf 100 ml Wasser gegossen und dann dreimal mit je 50 ml Ethylacetat extrahiert. Die vereinigten organischen Phasen wurden zweimal mit je 100 ml Wasser gewaschen, über Magnesiumsulfat getrocknet, filtriert und anschliessend eingeengt. Chromatographische Reinigung des Rückstandes an Kieselgel mit Hexan/Ethylacetat (Vol. 2:1) und zweimaliges Umkristallisieren der gemäss Dünnschichtchromatographie reinen Fraktionen aus Aceton ergab 0,3 g 1,4-Butylen bis[2,5-bis(4-[6-acryloyloxyhexyloxy)]phenylcarbonyloxy)benzoesäureester]; Smp. (C-N) 59°C, Klp. (N-I) 105°C.

Die als Ausgangsmaterial verwendete 2,5-bis(4-[6-Acryloyloxyhexyloxy)]phenylcarbonyloxy)benzoesäure wurde wie folgt hergestellt:
(a). Zu einer Lösung von 8,3 g 2,5 2,5-Dihydroxybenzaldehyd, 35,1 g 4-[6-Acryloyloxyhexyloxy]benzoesäure und 0,1 g 4-(Dimethylamino)pyridin (DMAP) in 600 ml Dichlormethan wurde unter Rühren bei 0°C innert 15 Minuten 29,7 g N,N '-Dicyclohexylcarbodiimid (DCC) gegeben. Das Reaktionsgemisch wurde über Nacht bei Raumtemperatur weitergerührt, dann filtriert, und anschliessend eingeengt. Chromatographische Reinigung des Rückstandes an Kieselgel mit Hexan/Ethylacetat (Vol. 1:1) und zweimaliges Umkristallisieren der gemäss Dünnschichtchromatographie reinen Fraktionen aus Ethylalkohol ergab 7,8 g 2,5-bis(4-[6-Acryloyloxyhexyloxy)]phenylcarbonyloxy)benzaldehyd.
(b). Eine Lösung von 3,9 g 2,5-bis(4-[6-Acryloyloxyhexyloxy)]phenylcarbonyloxy)benzaldehyd in 50 ml Aceton wurde tropfenweise mit 18 ml Jones' Reagenz versetzt. Das Gemisch wurde 1 Stunde bei Raumtemperatur gerührt, auf 100 ml Wasser gegossen und dann dreimal mit je 50 ml Ethylacetat extrahiert. Die vereinigten organischen Phasen wurden zweimal mit je 100 ml Wasser gewaschen, über Magnesiumsulfat getrocknet, filtriert und anschliessend eingeengt. Der Rückstand wurde aus tert.-Butyl-methylether bei -78°C umkristallisiert. Dies ergab 2,3 g 2,5-bis(4-[6-Acryloyloxyhexyloxy)]phenylcarbonyloxy)benzoesäure; Smp. (C-I) 126°C, Klp. (N-I) 122°C.

In analoger Weise können folgende Verbindungen hergestellt werden:
1,5-Pentylen bis[2,5-bis(4-[6-acryloyloxyhexyloxy)]phenylcarbonyloxy)-benzoesäureester]; Smp. (C-N) 62°C, Klp. (N-I) 96°C.
1,6-Hexylen bis[2,5-bis(4-[6-acryloyloxyhexyloxy)]phenylcarbonyloxy)benzoesäureester]; Smp. (C-N) 94°C, Klp. (N-I) 113°C.
1,7-Heptylen bis[2,5-bis(4-[6-acryloyloxyhexyloxy)]phenylcarbonyloxy)benzoesäureester]; Smp. (C-N) 45°C, Klp. (N-I) 85°C.
1,8-Octylen bis[2,5-bis(4-[6-acryloyloxyhexyloxy)]phenylcarbonyloxy)benzoesäureester]; Smp. (C-N) 65°C, Klp. (N-I) 97°C.
1,9-Nonylen bis[2,5-bis(4-[6-acryloyloxyhexyloxy)]phenylcarbonyloxy)benzoesäureester].
1,10-Decylen bis[2,5-bis(4-[6-acryloyloxyhexyloxy)]phenylcarbonyloxy)benzoesäureester].
1,4-Butylen bis[2,5-bis(4-[7-acryloyloxyheptyloxy)]phenylcarbonyloxy)benzoesäureester].
1,5-Pentylen bis[2,5-bis(4-[7-acryloyloxyheptyloxy)]phenylcarbonyloxy)benzoesäureester].
1,6-Hexylen bis[2,5-bis(4-[7-acryloyloxyheptyloxy)]phenylcarbonyloxy)benzoesäureester].
1,7-Heptylen bis[2,5-bis(4-[7-acryloyloxyheptyloxy)]phenylcarbonyloxy)benzoesäureester].
1,8-Octylen bis[2,5-bis(4-[7-acryloyloxyheptyloxy)]phenylcarbonyloxy)benzoesäureester].
1,9-Nonylen bis[2,5-bis(4-[7-acryloyloxyheptyloxy)]phenylcarbonyloxy)benzoesäureester].
1,10-Decylen bis[2,5-bis(4-[7-acryloyloxyheptyloxy)]phenylcarbonyloxy)benzoesäureester].
1,4-Butylen bis[2,5-bis(4-[8-acryloyloxyoctyloxy)]phenylcarbonyloxy)benzoesäureester].
1,5-Pentylen bis[2,5-bis(4-[8-acryloyloxyoctyloxy)]phenylcarbonyloxy)benzoesäureester].
1,6-Hexylen bis[2,5-bis(4-[8-acryloyloxyoctyloxy)]phenylcarbonyloxy)benzoesäureester].
1,7-Heptylen bis[2,5-bis(4-[8-acryloyloxyoctyloxy)]phenylcarbonyloxy)benzoesäureester].
1,8-Octylen bis[2,5-bis(4-[8-acryloyloxyoctyloxy)]phenylcarbonyloxy)benzoesäureester].
1,9-Nonylen bis[2,5-bis(4-[8-acryloyloxyoctyloxy)]phenylcarbonyloxy)benzoesäureester].
1,10-Decylen bis[2,5-bis(4-[8-acryloyloxyoctyloxy)]phenylcarbonyloxy)benzoesäureester].
1,4-Butylen bis[2,5-bis(4-[9-acryloyloxynonyloxy)]phenylcarbonyloxy)benzoesäureester].
1,5-Pentylen bis[2,5-bis(4-[9-acryloyloxynonyloxy)]phenylcarbonyloxy)benzoesäureester].
1,6-Hexylen bis[2,5-bis(4-[9-acryloyloxynonyloxy)]phenylcarbonyloxy)benzoesäureester].
1,7-Heptylen bis[2,5-bis(4-[9-acryloyloxynonyloxy)]phenylcarbonyloxy)benzoesäureester].
1,8-Octylen bis[2,5-bis(4-[9-acryloyloxynonyloxy)]phenylcarbonyloxy)benzoesäureester].
1,9-Nonylen bis[2,5-bis(4-[9-acryloyloxynonyloxy)]phenylcarbonyloxy)benzoesäureester].
1,10-Decylen bis[2,5-bis(4-[9-acryloyloxynonyloxy)]phenylcarbonyloxy)benzoesäureester].
1,4-Butylen bis[2,5-bis(4-[10-acryloyloxydecyloxy)]phenylcarbonyloxy)benzoesäureester].
1,5-Pentylen bis[2,5-bis(4-[10-acryloyloxydecyloxy)]phenylcarbonyloxy)benzoesäureester].
1,6-Hexylen bis[2,5-bis(4-[10-acryloyloxydecyloxy)]phenylcarbonyloxy)benzoesäureester].
1,7-Heptylen bis[2,5-bis(4-[10-acryloyloxydecyloxy)]phenylcarbonyloxy)benzoesäureester].
1,8-Octylen bis[2,5-bis(4-[10-acryloyloxydecyloxy)]phenylcarbonyloxy)benzoesäureester].
1,9-Nonylen bis[2,5-bis(4-[10-acryloyloxydecyloxy)]phenylcarbonyloxy)benzoesäureester].
1,10-Decylen bis[2,5-bis(4-[10-acryloyloxydecyloxy)]phenylcarbonyloxy)benzoesäureester].
1,4-Butylen bis[2,5-bis(4-[11-acryloyloxyundecyloxy)]phenylcarbonyloxy)benzoesäureester].
1,5-Pentylen bis[2,5-bis(4-[11-acryloyloxyundecyloxy)]phenylcarbonyloxy)benzoesäureester].
1,6-Hexylen bis[2,5-bis(4-[11-acryloyloxyundecyloxy)]phenylcarbonyloxy)benzoesäureester].
1,7-Heptylen bis[2,5-bis(4-[11-acryloyloxyundecyloxy)]phenylcarbonyloxy)benzoesäureester].
1,8-Octylen bis[2,5-bis(4-[11-acryloyloxyundecyloxy)]phenylcarbonyloxy)benzoesäureester].
1,9-Nonylen bis[2,5-bis(4-[11-acryloyloxyundecyloxy)]phenylcarbonyloxy)benzoesäureester].
1,10-Decylen bis[2,5-bis(4-[11-acryloyloxyundecyloxy)]phenylcarbonyloxy)benzoesäureester].
1,5-Pentylen bis[2,5-bis(2-fluor-4-[6-acryloyloxyhexyloxy)]phenylcarbonyloxy)benzoesäureester].
1,5-Pentylen bis[2,5-bis(2-chlor-4-[6-acryloyloxyhexyloxy)]phenylcarbonyloxy)benzoesäureester].
1,5-Pentylen bis[2,5-bis(2-brom-4-[6-acryloyloxyhexyloxy)]phenylcarbonyloxy)benzoesäureester].
1,5-Pentylen bis[2,5-bis(2-cyano-4-[6-acryloyloxyhexyloxy)]phenylcarbonyloxy)benzoesäureester].
1,5-Pentylen bis[2,5-bis(2,3-difluor-4-[6-acryloyloxyhexyloxy)]phenylcarbonyloxy)benzoesäureester].
1,5-Pentylen bis[2,5-bis(2,3-dichlor-4-[6-acryloyloxyhexyloxy)]phenylcarbonyloxy)benzoesäureester].
1,5-Pentylen bis[2,5-bis(4-[6-methacryloyloxyhexyloxy)]phenylcarbonyloxy)benzoesäureester].
1,6-Hexylen bis[2,5-bis(4-[6-methacryloyloxyhexyloxy)]phenylcarbonyloxy)benzoesäureester].
1,7-Heptylen bis[2,5-bis(4-[6-methacryloyloxyhexyloxy)]phenylcarbonyloxy)benzoesäureester].
1,8-Octylen bis[2,5-bis(4-[6-methacryloyloxyhexyloxy)]phenylcarbonyloxy)benzoesäureester].
1,9-Nonylen bis[2,5-bis(4-[6-methacryloyloxyhexyloxy)]phenylcarbonyloxy)benzoesäureester].
1,10-Decylen bis[2,5-bis(4-[6-methacryloyloxyhexyloxy)]phenylcarbonyloxy)benzoesäureester].
1,5-Pentylen bis[2,5-bis(2,3-dichlor-4-[6-vinyloxyhexyloxy)]phenylcarbonyloxy)benzoesäureester].
1,5-Pentylen bis[2,5-bis(4-[6-vinyloxyhexyloxy)]phenylcarbonyloxy)benzoesäureester].
1,6-Hexylen bis[2,5-bis(4-[6-vinyloxyhexyloxy)]phenylcarbonyloxy)benzoesäureester].
1,7-Heptylen bis[2,5-bis(4-[6-vinyloxyhexyloxy)]phenylcarbonyloxy)benzoesäureester].
1,8-Octylen bis[2,5-bis(4-[6-vinyloxyhexyloxy)]phenylcarbonyloxy)benzoesäureester].
1,9-Nonylen bis[2,5-bis(4-[6-vinyloxyhexyloxy)]phenylcarbonyloxy)benzoesäureester].
1,10-Decylen bis[2,5-bis(4-[6-vinyloxyhexyloxy)]phenylcarbonyloxy)benzoesäureester].
1,5-Pentylen bis[2,5-bis(2,3-dichlor-4-[5,6-epoxyhexyloxy)]phenylcarbonyloxy)benzoesäureester].
1,5-Pentylen bis[2,5-bis(4-[5,6-epoxyhexyloxy)]phenylcarbonyloxy)benzoesäureester].
1,6-Hexylen bis[2,5-bis(4-[5,6-epoxyhexyloxy)]phenylcarbonyloxy)benzoesäureester].
1,7-Heptylen bis[2,5-bis(4-[5,6-epoxyhexyloxy)]phenylcarbonyloxy)benzoesäureester].
1,8-Octylen bis[2,5-bis(4-[5,6-epoxyhexyloxy)]phenylcarbonyloxy)benzoesäureester].
1,9-Nonylen bis[2,5-bis(4-[5,6-epoxyhexyloxy)]phenylcarbonyloxy)benzoesäureester].
1,10-Decylen bis[2,5-bis(4-[5,6-epoxyhexyloxy)]phenylcarbonyloxy)benzoesäureester].

### Beispiel 2

(a). Zu einer Lösung von 4,0 g Hydrochinon, 35,1 g 2,5-bis(4-[6-Acryloyloxyhexyloxy)]phenylcarbonyloxy)benzoesäure und 0,1 g 4-(Dimethylamino)pyridin in 600 ml Dichlormethan wird unter Rühren bei 0°C innert 15 Minuten 29,7 g N,N '-Dicyclohexylcarbodiimid gegeben. Das Reaktionsgemisch wird über Nacht bei Raumtemperatur weitergerührt, dann filtriert und anschliessend eingeengt. Chromatographische Reinigung des Rückstandes an Kieselgel mit Hexan/Ethylacetat (Vol. 1:1) und zweimaliges Umkristallisieren der gemäss Dünnschichtchromatographie reinen Fraktionen aus Ethylalkohol ergibt 7,8 g 1,4-Phenylen bis[2,5-bis(4-[6-acryloyloxyhexyloxy)]phenylcarbonyloxy)benzoesäureester].

In analoger Weise können folgende Verbindungen hergestellt werden:
1,4-Phenylen bis[2,5-bis(4-[7-acryloyloxyheptyloxy)]phenylcarbonyloxy)benzoesäureester].
1,4-Phenylen bis[2,5-bis(4-[8-acryloyloxyoctyloxy)]phenylcarbonyloxy)benzoesäureester].
1,4-Phenylen bis[2,5-bis(4-[9-acryloyloxynonyloxy)]phenylcarbonyloxy)benzoesäureester].
1,4-Phenylen bis[2,5-bis(4-[10-acryloyloxydecyloxy)]phenylcarbonyloxy)benzoesäureester].
1,4-Phenylen bis[2,5-bis(4-[11-acryloyloxyundecyloxy)]phenylcarbonyloxy)benzoesäureester].
trans-1,4-Cyclohexylen bis[2,5-bis(4-[6-acryloyloxyhexyloxy)]phenylcarbonyloxy)benzoesäureester].
trans-1,4-Cyclohexylen bis[2,5-bis(4-[7-acryloyloxyheptyloxy)]phenylcarbonyloxy)benzoesäureester].
trans-1,4-Cyclohexylen bis[2,5-bis(4-[8-acryloyloxyoctyloxy)]phenylcarbonyloxy)benzoesäureester].
trans-1,4-Cyclohexylen bis[2,5-bis(4-[9-acryloyloxynonyloxy)]phenylcarbonyloxy)benzoesäureester].
trans-1,4-Cyclohexylen bis[2,5-bis(4-[10-acryloyloxydecyloxy)]phenylcarbonyloxy)benzoesäureester].
trans-1,4-Cyclohexylen bis[2,5-bis(4-[11-acryloyloxyundecyloxy)]phenylcarbonyloxy)benzoesäureester].
4,4'-Biphenylen bis[2,5-bis(4-[6-acryloyloxyhexyloxy)]phenylcarbonyloxy)benzoesäureester].
4,4'-Biphenylen bis[2,5-bis(4-[7-acryloyloxyheptyloxy)]phenylcarbonyloxy)benzoesäureester].
4,4'-Biphenylen bis[2,5-bis(4-[8-acryloyloxyoctyloxy)]phenylcarbonyloxy)benzoesäureester].
4,4'-Biphenylen bis[2,5-bis(4-[9-acryloyloxynonyloxy)]phenylcarbonyloxy)benzoesäureester].
4,4'-Biphenylen bis[2,5-bis(4-[10-acryloyloxydecyloxy)]phenylcarbonyloxy)benzoesäureester].
4,4'-Biphenylen bis[2,5-bis(4-[11-acryloyloxyundecyloxy)]phenylcarbonyloxy)benzoesäureester].
1,4-Phenylen bis[2,5-bis(4-[6-methacryloyloxyhexyloxy)]phenylcarbonyloxy)benzoesäureester].
1,4-Phenylen bis[2,5-bis(4-[7-methacryloyloxyheptyloxy)]phenylcarbonyloxy)benzoesäureester].
1,4-Phenylen bis[2,5-bis(4-[8-methacryloyloxyoctyloxy)]phenylcarbonyloxy)benzoesäureester].
1,4-Phenylen bis[2,5-bis(4-[9-methacryloyloxynonyloxy)]phenylcarbonyloxy)benzoesäureester].
1,4-Phenylen bis[2,5-bis(4-[10-methacryloyloxydecyloxy)]phenylcarbonyloxy)benzoesäureester].
1,4-Phenylen bis[2,5-bis(4-[11-methacryloyloxyundecyloxy)]phenylcarbonyloxy)benzoesäureester].
1,4-Phenylen bis[2,5-bis(2,3-dichlor-4-[6-vinyloxyhexyloxy)]phenylcarbonyloxy)benzoesäureester].
1,4-Phenylen bis[2,5-bis(4-[6-vinyloxyhexyloxy)]phenylcarbonyloxy)benzoesäureester].
1,4-Phenylen bis[2,5-bis(4-[7-vinyloxyheptyloxy)]phenylcarbonyloxy)benzoesäureester].
1,4-Phenylen bis[2,5-bis(4-[8-acryloyloxyoctyloxy)]phenylcarbonyloxy)benzoesäureester].
1,4-Phenylen bis[2,5-bis(4-[9-vinyloxynonyloxy)]phenylcarbonyloxy)benzoesäureester].
1,4-Phenylen bis[2,5-bis(4-[10-vinyloxydecyloxy)]phenylcarbonyloxy)benzoesäureester].
1,4-Phenylen bis[2,5-bis(4-[11-vinyloxyundecyloxy)]phenylcarbonyloxy)benzoesäureester].
1,4-Phenylen bis[2,5-bis(2,3-dichlor-4-[5,6-epoxyhexyloxy)]phenylcarbonyloxy)benzoesäureester].
1,4-Phenylen bis[2,5-bis(4-[5,6-epoxyhexyloxy)]phenylcarbonyloxy)benzoesäureester].
1,4-Phenylen bis[2,5-bis(4-[6,7-epoxyheptyloxy)]phenylcarbonyloxy)benzoesäureester].
1,4-Phenylen bis[2,5-bis(4-[7,8-epoxyoctyloxy)]phenylcarbonyloxy)benzoesäureester].
1,4-Phenylen bis[2,5-bis(4-[8,9-epoxynonyloxy)]phenylcarbonyloxy)benzoesäureester].
1,4-Phenylen bis[2,5-bis(4-[9,10-epoxydecyloxy)]phenylcarbonyloxy)benzoesäureester].
1,4-Phenylen bis[2,5-bis(4-[10,11-epoxyundecyloxy)]phenylcarbonyloxy)benzoesäureester].

### Beispiel 3

Zu einer Lösung von 0,7 g 2,5-bis(4-[6-acryloyloxyhexyloxy)]phenylcarbonyloxy)benzylalkohol und 0,11 g 1,4-Dibrombutan in 20 ml Dimethylsulfoxid wird unter Rühren bei Raumtemperatur 0,15 g Kalium-tert.-butoxid gegeben. Das Reaktionsgemisch wird über Nacht bei Raumtemperatur gerührt, auf 100 ml Wasser gegossen und dann dreimal mit je 50 ml Ethylacetat extrahiert. Die vereinigten organischen Phasen werden zweimal mit je 100 ml Wasser gewaschen, über Magnesiumsulfat getrocknet, filtriert und anschliessend eingeengt. Chromatographische Reinigung des Rückstandes an Kieselgel mit Hexan/Ethylacetat (Vol. 2:1) und zweimaliges Umkristallisieren der gemäss Dünnschichtchromatographie reinen Fraktionen aus Aceton ergibt 0,3 g 1,4-Butylen bis[2,5-bis(4-[6-acryloyloxyhexyloxy)]phenylcarbonyloxy)benzylether].

Der als Ausgangsmaterial verwendete 2,5-bis(4-[6-Acryloyloxyhexyloxy)]phenylcarbonyloxy)benzylalkohol wird wie folgt hergestellt:

(a). Ein Gemisch von 32 g Natriumborhydrid und 180 ml Ethanol wird bei 0°C mit 165 ml Wasser versetzt, 10 Minuten weitergerührt, dann tropfenweise bei 0-5°C mit einer Lösung von 50 g 2,5-bis(4-[6-Acryloxyhexyloxy)]phenylcarbonyloxy)benzaldehyd in 100 ml Ethanol und 10 ml Dichlormethan versetzt. Das Reaktionsgemisch wird weitere 30 Minuten bei 0°C gerührt, dann auf 100 ml Dichlormethan gegossen und zweimal mit je 50 ml Dichlormethan extrahiert. Die vereinigten organischen Phasen werden dann zweimal mit je 100 ml Wasser gewaschen, über Magnesiumsulfat getrocknet, filtriert und das Filtrat eingeengt. Der Rückstand wird aus tert.-Butyl-methylether umkristallisiert. Dies ergibt 35 g 2,5-bis(4-[6-Acryloyloxyhexyloxy)]phenylcarbonyloxy)benzylalkohol.

In analoger Weise können folgende Verbindungen hergestellt werden:
1,5-Pentylen bis[2,5-bis(4-[6-acryloyloxyhexyloxy)]phenylcarbonyloxy)benzylether].
1,6-Hexylen bis[2,5-bis(4-[6-acryloyloxyhexyloxy)]phenylcarbonyloxy)benzylether].
1,7-Heptylen bis[2,5-bis(4-[6-acryloyloxyhexyloxy)]phenylcarbonyloxy)benzylether].
1,8-Octylen bis[2,5-bis(4-[6-acryloyloxyhexyloxy)]phenylcarbonyloxy)benzylether].
1,9-Nonylen bis[2,5-bis(4-[6-acryloyloxyhexyloxy)]phenylcarbonyloxy)benzylether].
1,10-Decylen bis[2,5-bis(4-[6-acryloyloxyhexyloxy)]phenylcarbonyloxy)benzylether].
1,4-Butylen bis[2,5-bis(4-[7-acryloyloxyheptyloxy)]phenylcarbonyloxy)benzylether].
1,5-Pentylen bis[2,5-bis(4-[7-acryloyloxyheptyloxy)]phenylcarbonyloxy)benzylether].
1,6-Hexylen bis[2,5-bis(4-[7-acryloyloxyheptyloxy)]phenylcarbonyloxy)benzylether].
1,7-Heptylen bis[2,5-bis(4-[7-acryloyloxyheptyloxy)]phenylcarbonyloxy)benzylether].
1,8-Octylen bis[2,5-bis(4-[7-acryloyloxyheptyloxy)]phenylcarbonyloxy)benzylether].
1,9-Nonylen bis[2,5-bis(4-[7-acryloyloxyheptyloxy)]phenylcarbonyloxy)benzylether].
1,10-Decylen bis[2,5-bis(4-[7-acryloyloxyheptyloxy)]phenylcarbonyloxy)benzylether].
1,4-Butylen bis[2,5-bis(4-[8-acryloyloxyoctyloxy)]phenylcarbonyloxy)benzylether].
1,5-Pentylen bis[2,5-bis(4-[8-acryloyloxyoctyloxy)]phenylcarbonyloxy)benzylether].
1,6-Hexylen bis[2,5-bis(4-[8-acryloyloxyoctyloxy)]phenylcarbonyloxy)benzylether].
1,7-Heptylen bis[2,5-bis(4-[8-acryloyloxyoctyloxy)]phenylcarbonyloxy)benzylether].
1,8-Octylen bis[2,5-bis(4-[8-acryloyloxyoctyloxy)]phenylcarbonyloxy)benzylether].
1,9-Nonylen bis[2,5-bis(4-[8-acryloyloxyoctyloxy)]phenylcarbonyloxy)benzylether].
1,10-Decylen bis[2,5-bis(4-[8-acryloyloxyoctyloxy)]phenylcarbonyloxy)benzylether].
1,4-Butylen bis[2,5-bis(4-[9-acryloyloxynonyloxy)]phenylcarbonyloxy)benzylether].
1,5-Pentylen bis[2,5-bis(4-[9-acryloyloxynonyloxy)]phenylcarbonyloxy)benzylether].
1,6-Hexylen bis[2,5-bis(4-[9-acryloyloxynonyloxy)]phenylcarbonyloxy)benzylether].
1,7-Heptylen bis[2,5-bis(4-[9-acryloyloxynonyloxy)]phenylcarbonyloxy)benzylether].
1,8-Octylen bis[2,5-bis(4-[9-acryloyloxynonyloxy)]phenylcarbonyloxy)benzylether].
1,9-Nonylen bis[2,5-bis(4-[9-acryloyloxynonyloxy)]phenylcarbonyloxy)benzylether].
1,10-Decylen bis[2,5-bis(4-[9-acryloyloxynonyloxy)]phenylcarbonyloxy)benzylether].
1,4-Butylen bis[2,5-bis(4-[10-acryloyloxydecyloxy)]phenylcarbonyloxy)benzylether].
1,5-Pentylen bis[2,5-bis(4-[10-acryloyloxydecyloxy)]phenylcarbonyloxy)benzylether].
1,6-Hexylen bis[2,5-bis(4-[10-acryloyloxydecyloxy)]phenylcarbonyloxy)benzylether].
1,7-Heptylen bis[2,5-bis(4-[10-acryloyloxydecyloxy)]phenylcarbonyloxy)benzylether].
1,8-Octylen bis[2,5-bis(4-[10-acryloyloxydecyloxy)]phenylcarbonyloxy)benzylether].
1,9-Nonylen bis[2,5-bis(4-[10-acryloyloxydecyloxy)]phenylcarbonyloxy)benzylether].
1,10-Decylen bis[2,5-bis(4-[10-acryloyloxydecyloxy)]phenylcarbonyloxy)benzylether].
1,4-Butylen bis[2,5-bis(4-[11-acryloyloxyundecyloxy)]phenylcarbonyloxy)benzylether].
1,5-Pentylen bis[2,5-bis(4-[11-acryloyloxyundecyloxy)]phenylcarbonyloxy)benzylether].
1,6-Hexylen bis[2,5-bis(4-[11-acryloyloxyundecyloxy)]phenylcarbonyloxy)benzylether].
1,7-Heptylen bis[2,5-bis(4-[11-acryloyloxyundecyloxy)]phenylcarbonyloxy)benzylether].
1,6-Hexylen bis[2,5-bis(4-[11-acryloyloxyundecyloxy)]phenylcarbonyloxy)benzylether].
1,9-Nonylen bis[2,5-bis(4-[11-acryloyloxyundecyloxy)]phenylcarbonyloxy)benzylether].
1,10-Decylen bis[2,5-bis(4-[11-acryloyloxyundecyloxy)]phenylcarbonyloxy)benzylether].
1,5-Pentylen bis[2,5-bis(4-[6-methacryloyloxyhexyloxy)]phenylcarbonyloxy)benzylether].
1,6-Hexylen bis[2,5-bis(4-[6-methacryloyloxyhexyloxy)]phenylcarbonyloxy)benzylether].
1,7-Heptylen bis[2,5-bis(4-[6-methacryloyloxyhexyloxy)]phenylcarbonyloxy)benzylether].
1,8-Octylen bis[2,5-bis(4-[6-methacryloyloxyhexyloxy)]phenylcarbonyloxy)benzylether].
1,9-Nonylen bis[2,5-bis(4-[6-methacryloyloxyhexyloxy)]phenylcarbonyloxy)benzylether].
1,10-Decylen bis[2,5-bis(4-[6-methacryloyloxyhexyloxy)]phenylcarbonyloxy)benzylether].
1,5-Pentylen bis[2,5-bis(4-[6-vinyloxyhexyloxy)]phenylcarbonyloxy)benzylether].
1,6-Hexylen bis[2,5-bis(4-[6-vinyloyloxyhexyloxy)]phenylcarbonyloxy)benzylether].
1,7-Heptylen bis[2,5-bis(4-[6-vinyloyloxyhexyloxy)]phenylcarbonyloxy)benzylether].
1,8-Octylen bis[2,5-bis(4-[6-vinyloyloxyhexyloxy)]phenylcarbonyloxy)benzylether].
1,9-Nonylen bis[2,5-bis(4-[6-vinyloyloxyhexyloxy)]phenylcarbonyloxy)benzylether].
1,10-Decylen bis[2,5-bis(4-[6-vinyloyloxyhexyloxy)]phenylcarbonyloxy)benzylether].

### Beispiel 4

Zu einer Lösung von 0,11 g 1,4-Diaminobutan in 20 ml Dichlormethan wird unter Rühren bei Raumtemperatur eine Lösung von 0,7 g 2,5-bis(4-[6-Acryloyloxyhexyloxy)]phenylcarbonyloxy)benzoesäurechlorid und 20 ml Dichlormethan tropfenweise dazugegeben. Das Reaktionsgemisch wird über Nacht bei Raumtemperatur gerührt, auf 100 ml Wasser gegossen und dann dreimal mit je 50 ml Ethylacetat extrahiert. Die vereinigten organischen Phasen werden zweimal mit je 100 ml Wasser gewaschen, über Magnesiumsulfat getrocknet, filtriert und anschliessend eingeengt. Chromatographische Reinigung des Rückstandes an Kieselgel mit Hexan/Ethylacetat (Vol. 2:1) und zweimaliges Umkristallisieren der gemäss Dünnschichtchromatographie reinen Fraktionen aus Aceton ergibt 0,3 g 1,4-Butylen bis[2,5-bis(4-[6-acryloyloxyhexyloxy)]phenylcarbonyloxy)benzamid].

Das als Ausgangsmaterial verwendete 2,5-bis(4-[6-Acryloyloxyhexyloxy)]phenylcarbonyloxy)benzoesäurechlorid wird wie folgt hergestellt:

(a). 1,5 g 2,5-bis(4-[6-Acryloyloxyhexyloxy)]phenylcarbonyloxy)benzoesäure werden mit 25 ml Thionylchlorid in 100 ml Toluol 2 Stunden bei 80°C erwärmt. Die erhaltene Lösung wird unter vermindertem Druck eingedamft, der Rückstand mit 20 ml absolutem Toluol versetzt und die Lösung nochmals unter vermindertem Druck eingedamft. Der Rückstand wird ohne weitere Reinigung in die nächste Stufe eingesetzt.

In analoger Weise können folgende Verbindungen hergestellt werden:
1,5-Pentylen bis[2,5-bis(4-[6-acryloyloxyhexyloxy)]phenylcarbonyloxy)benzamid].
1,6-Hexylen bis[2,5-bis(4-[6-acryloxyhexyloxy)]phenylcarbonyloxy)benzamid].
1,7-Heptylen bis[2,5-bis(4-[6-acryloyloxyhexyloxy)]phenylcarbonyloxy)benzamid].
1,8-Octylen bis[2,5-bis(4-[6-acryloyloxyhexyloxy)]phenylcarbonyloxy)benzamid].
1,9-Nonylen bis[2,5-bis(4-[6-acryloyloxyhexyloxy)]phenylcarbonyloxy)benzamid].
1,10-Decylen bis[2,5-bis(4-[6-acryloyloxyhexyloxy)]phenylcarbonyloxy)benzamid].
1,4-Butylen bis[2,5-bis(4-[7-acryloyloxyheptyloxy)]phenylcarbonyloxy)benzamid].
1,5-Pentylen bis[2,5-bis(4-[7-acryloyloxyheptyloxy)]phenylcarbonyloxy)benzamid].
1,6-Hexylen bis[2,5-bis(4-[7-acryloyloxyheptyloxy)]phenylcarbonyloxy)benzamid].
1,7-Heptylen bis[2,5-bis(4-[7-acryloxyheptyloxy)]phenylcarbonyloxy)benzamid].
1,8-Octylen bis[2,5-bis(4-[7-acryloxyheptyloxy)]phenylcarbonyloxy)benzamid].
1,9-Nonylen bis[2,5-bis(4-[7-acryloxyheptyloxy)]phenylcarbonyloxy)benzamid].
1,10-Decylen bis[2,5-bis(4-[7-acryloxyheptyloxy)]phenylcarbonyloxy)benzamid].
1,4-Butylen bis[2,5-bis(4-[8-acryloyloxyoctyloxy)]phenylcarbonyloxy)benzamid].
1,5-Pentylen bis[2,5-bis(4-[8-acryloyloxyoctyloxy)]phenylcarbonyloxy)benzamid].
1,6-Hexylen bis[2,5-bis(4-[8-acryloyloxyoctyloxy)]phenylcarbonyloxy)benzamid].
1,7-Heptylen bis[2,5-bis(4-[8-acryloyloxyoctyloxy)]phenylcarbonyloxy)benzamid].
1,8-Octylen bis[2,5-bis(4-[8-acryloyloxyoctyloxy)]phenylcarbonyloxy)benzamid].
1,9-Nonylen bis[2,5-bis(4-[8-acryloyloxyoctyloxy)]phenylcarbonyloxy)benzamid].
1,10-Decylen bis[2,5-bis(4-[8-acryloyloxyoctyloxy)]phenylcarbonyloxy)benzamid].
1,4-Butylen bis[2,5-bis(4-[9-acryloyloxynonyloxy)]phenylcarbonyloxy)benzamid].
1,5-Pentylen bis[2,5-bis(4-[9-acryloyloxynonyloxy)]phenylcarbonyloxy)benzamid].
1,6-Hexylen bis[2,5-bis(4-[9-acryloyloxynonyloxy)]phenylcarbonyloxy)benzamid].
1,7-Heptylen bis[2,5-bis(4-[9-acryloyloxynonyloxy)]phenylcarbonyloxy)benzamid].
1,8-Octylen bis[2,5-bis(4-[9-acryloyloxynonyloxy)]phenylcarbonyloxy)benzamid].
1,9-Nonylen bis[2,5-bis(4-[9-acryloyloxynonyloxy)]phenylcarbonyloxy)benzamid].
1,10-Decylen bis[2,5-bis(4-[9-acryloyloxynonyloxy)]phenylcarbonyloxy)benzamid].
1,4-Butylen bis[2,5-bis(4-[10-acryloyloxydecyloxy)]phenylcarbonyloxy)benzamid].
1,5-Pentylen bis[2,5-bis(4-[10-acryloyloxydecyloxy)]phenylcarbonyloxy)benzamid].
1,6-Hexylen bis[2,5-bis(4-[10-acryloyloxydecyloxy)]phenylcarbonyloxy)benzamid].
1,7-Heptylen bis[2,5-bis(4-[10-acryloyloxydecyloxy)]phenylcarbonyloxy)benzamid].
1,8-Octylen bis[2,5-bis(4-[10-acryloyloxydecyloxy)]phenylcarbonyloxy)benzamid].
1,9-Nonylen bis[2,5-bis(4-[10-acryloyloxydecyloxy)]phenylcarbonyloxy)benzamid].
1,10-Decylen bis[2,5-bis(4-[10-acryloyloxydecyloxy)]phenylcarbonyloxy)benzamid].
1,4-Butylen bis[2,5-bis(4-[11-acryloyloxyundecyloxy)]phenylcarbonyloxy)benzamid].
1,5-Pentylen bis[2,5-bis(4-[11-acryloyloxyundecyloxy)]phenylcarbonyloxy)benzamid].
1,6-Hexylen bis[2,5-bis(4-[11-acryloyloxyundecyloxy)]phenylcarbonyloxy)benzamid].
1,7-Heptylen bis[2,5-bis(4-[11-acryloyloxyundecyloxy)]phenylcarbonyloxy)benzamid].
1,8-Octylen bis[2,5-bis(4-[11-acryloyloxyundecyloxy)]phenylcarbonyloxy)benzamid].
1,9-Nonylen bis[2,5-bis(4-[11-acryloyloxyundecyloxy)]phenylcarbonyloxy)benzamid].
1,10-Decylen bis[2,5-bis(4-[11-acryloyloxyundecyloxy)]phenylcarbonyloxy)benzamid].

### Beispiel 5

(a). Zu einer Lösung von 4,0 g 1,5-bis(hydrochinoyl)pentan, 35,1 g 4-[6-Acryloyloxyhexyloxy]benzosäure und 0,1 g 4-(Dimethylamino)pyridin in 600 ml Dichlormethan wird unter Rühren bei 0°C innert 15 Minuten 29,7 g N,N'-Dicyclohexylcarbodiimid gegeben. Das Reaktionsgemisch wird über Nacht bei Raumtemperatur weitergerührt, dann filtriert und anschliessend eingeengt. Chromatographische Reinigung des Rückstandes an Kieselgel mit Hexan/Ethylacetat (Vol. 1:1) und zweimaliges Umkristallisieren der gemäss Dünnschichtchromatographie reinen Fraktionen aus Ethylalkohol ergibt 7,8 g 1,5-bis[2,5-bis(4-[6-Acryloyloxyhexyloxy)]phenylcarbonyloxy)phenyl]pentan.

In analoger Weise können folgende Verbindungen hergestellt werden:
1,6-bis[2,5-bis(4-[6-Acryloyloxyhexyloxy)]phenylcarbonyloxy)phenyl]hexan.
1,7-bis[2,5-bis(4-[6-Acryloyloxyhexyloxy)]phenylcarbonyloxy)phenyl]heptan.
1,8-bis[2,5-bis(4-[6-Acryloyloxyhexyloxy)]phenylcarbonyloxy)phenyl]octan.
1,9-bis[2,5-bis(4-[6-Acryloyloxyhexyloxy)]phenylcarbonyloxy)phenyl]nonan.
1,10-bis[2,5-bis(4-[6-Acryloyloxyhexyloxy)]phenylcarbonyloxy)phenyl]decan.
1,6-bis[2,5-bis(4-[7-Acryloyloxyheptyloxy)]phenylcarbonyloxy)phenyl]hexan.
1,7-bis[2,5-bis(4-[7-Acryloyloxyheptyloxy)]phenylcarbonyloxy)phenyl]heptan.
1,8-bis[2,5-bis(4-[7-Acryloyloxyheptyloxy)]phenylcarbonyloxy)phenyl]octan.
1,9-bis[2,5-bis(4-[7-Acryloyloxyheptyloxy)]phenylcarbonyloxy)phenyl]nonan.
1,10-bis[2,5-bis(4-[7-Acryloyloxyheptyloxy)]phenylcarbonyloxy)phenyl]decan.
1,6-bis[2,5-bis(4-[8-Acryloyloxyoctyloxy)]phenylcarbonyloxy)phenyl]hexan.
1,7-bis[2,5-bis(4-[8-Acryloyloxyoctyloxy)]phenylcarbonyloxy)phenyl]heptan.
1,8-bis[2,5-bis(4-[8-Acryloyloxyoctyloxy)]phenylcarbonyloxy)phenyl]octan.
1,9-bis[2,5-bis(4-[8-Acryloyloxyoctyloxy)]phenylcarbonyloxy)phenyl]nonan.
1,10-bis[2,5-bis(4-[8-Acryloyloxyoctyloxy)]phenylcarbonyloxy)phenyl]decan.
1,6-bis[2,5-bis(4-[9-Acryloyloxynonyloxy)]phenylcarbonyloxy)phenyl]hexan.
1,7-bis[2,5-bis(4-[9-Acryloyloxynonyloxy)]phenylcarbonyloxy)phenyl]heptan.
1,8-bis[2,5-bis(4-[9-Acryloyloxynonyloxy)]phenylcarbonyloxy)phenyl]octan.
1,9-bis[2,5-bis(4-[9-Acryloyloxynonyloxy)]phenylcarbonyloxy)phenyl]nonan.
1,10-bis[2,5-bis(4-[9-Acryloyloxynonyloxy)]phenylcarbonyloxy)phenyl]decan.
1,6-bis[2,5-bis(4-[10-Acryloyloxydecyloxy)]phenylcarbonyloxy)phenyl]hexan.
1,7-bis[2,5-bis(4-[10-Acryloyloxydecyloxy)]phenylcarbonyloxy)phenyl]heptan.
1,8-bis[2,5-bis(4-[10-Acryloyloxydecyloxy)]phenylcarbonyloxy)phenyl]octan.
1,9-bis[2,5-bis(4-[10-Acryloyloxydecyloxy)]phenylcarbonyloxy)phenyl]nonan.
1,10-bis[2,5-bis(4-[10-Acryloyloxydecyloxy)]phenylcarbonyloxy)phenyl]decan.
1,6-bis[2,5-bis(4-[11-Acryloyloxyundecyloxy)]phenylcarbonyloxy)phenyl]hexan.
1,7-bis[2,5-bis(4-[11-Acryloyloxyundecyloxy)]phenylcarbonyloxy)phenyl]heptan.
1,8-bis[2,5-bis(4-[11-Acryloyloxyundecyloxy)]phenylcarbonyloxy)phenyl]octan.
1,9-bis[2,5-bis(4-[11-Acryloyloxyundecyloxy)]phenylcarbonyloxy)phenyl]nonan.
1,10-bis[2,5-bis(4-[11-Acryloyloxyundecyloxy)]phenylcarbonyloxy)phenyl]decan.
1,6-bis[2,5-bis(4-[6-Methacryloyloxyhexyloxy)]phenylcarbonyloxy)phenyl]hexan.
1,7-bis[2,5-bis(4-[6-Methacryloyloxyhexyloxy)]phenylcarbonyloxy)phenyl]heptan.
1,8-bis[2,5-bis(4-[6-Methacryloyloxyhexyloxy)]phenylcarbonyloxy)phenyl]octan.
1,9-bis[2,5-bis(4-[6-Methacryloyloxyhexyloxy)]phenylcarbonyloxy)phenyl]nonan.
1,10-bis[2,5-bis(4-[6-Methacryloyloxyhexyloxy)]phenylcarbonyloxy)phenyl]decan.
1,6-bis[2,5-bis(4-[6-Acryloyloxyhexyloxy)]phenylcarbonyloxy)phenyl]hexan.
1,7-bis[2,5-bis(4-[6-Vinyloxyhexyloxy)]phenylcarbonyloxy)phenyl]heptan.
1,8-bis[2,5-bis(4-[6-Vinyloxyhexyloxy)]phenylcarbonyloxy)phenyl]octan.
1,9-bis[2,5-bis(4-[6-Vinyloxyhexyloxy)]phenylcarbonyloxy)phenyl]nonan.
1,10-bis[2,5-bis(4-[6-Vinyloxyhexyloxy)]phenylcarbonyloxy)phenyl]decan.
1,6-bis[2,5-bis(4-[5,6-Epoxyhexyloxy)]phenylcarbonyloxy)phenyl]hexan.
1,7-bis[2,5-bis(4-[5,6-Epoxyhexyloxy)]phenylcarbonyloxy)phenyl]heptan.
1,8-bis[2,5-bis(4-[5,6-Epoxyhexyloxy)]phenylcarbonyloxy)phenyl]octan.
1,9-bis[2,5-bis(4-[5,6-Epoxyhexyloxy)]phenylcarbonyloxy)phenyl]nonan.
1,10-bis[2,5-bis(4-[5,6-Epoxyhexyloxy)]phenylcarbonyloxy)phenyl]decan.

### Beispiel 6

Zu einer Lösung von 0,7 g 2,5-bis(4-[6-Acryloyloxyhexyloxy)]phenylcarbonyloxy)benzaldehyd, 0,15 g 1,5-Diaminopentan in 20 ml Ethylalkohol wird unter Rühren und leichtem Rückfluss 5 Tropfen Eisessig dazu gegeben. Nach 10 Minuten wird das Reaktionsgemisch eingeengt. Chromatographische Reinigung des Rückstandes an Kieselgel mit Hexan/Ethylacetat (Vol. 2:1) und zweimaliges Umkristallisieren der gemäss Dünnschichtchromatographie reinen Fraktionen aus Aceton ergibt 0,3 g 1,5-bis[2,5-bis(4-[6-acryloyloxyhexyloxy)]phenylcarbonyloxy)benzyliden]pentyldiamin.

In analoger Weise können folgende Verbindungen hergestellt werden:
1,6-bis[2,5-bis(4-[6-acryloyloxyhexyloxy)]phenylcarbonyloxy)benzyliden]hexyldiamin.
1,7-bis[2,5-bis(4-[6-acryloyloxyhexyloxy)]phenylcarbonyloxy)benzyliden]heptyldiamin.
1,8-bis[2,5-bis(4-[6-acryloyloxyhexyloxy)]phenylcarbonyloxy)benzyliden]octyldiamin.
1,9-bis[2,5-bis(4-[6-acryloyloxyhexyloxy)]phenylcarbonyloxy)benzyliden]nonyldiamin.
1,10-bis[2,5-bis(4-[6-acryloyloxyhexyloxy)]phenylcarbonyloxy)benzyliden]decyldiamin.

### Beispiel 7

1,6-Hexylen bis[2,5-bis(4-[6-acryloyloxyhexyloxy)]phenylcarbonyloxy)benzoesäureester] wurde vorgelegt, mit 1 Gew.% eines Photoinitiators (IRGACURE, Ciba Geigy) und 1 Gew.% BHT versetzt, in Anisol gelöst (2 Gew.%) und dann bei 3500 Umdrehungen pro Minute auf eine mit PVA-beschichtete (PVA = Polyvinylalkohol) und geriebene Glasplatte geschleudert. Die Schicht wurde bei 90°C auf einer Wärmebank getrocknet, dann im Vakuumschank unter Vakuum bei 90°C mit Xenon-licht belichtet (zB. 3 Minuten). Eine parallel orientierte nematische Schicht mit einer Schichtdicke von 200µm und mit einer Doppelbrechung (Δn) von 0,15 wurde erzeugt. Diese Schicht fungiert als ein optischer Retarder.

### Beispiel 8

1,6-Hexylen bis[2,5-bis(4-[6-acryloyloxyhexyloxy)]phenylcarbonyloxy)benzoesäureester] wurde vorgelegt, mit 1 Gew.% IRGACURE und 1 Gew.% BHT versetzt, in Anisol gelöst (2 Gew.%) und dann bei 3500 Umdrehungen pro Minute auf eine Glasplatte geschleudert. Die Glasplatte wurde zuvor mit Methacryloyloxyethyl-3-(E)-[4-cyano-4'-biphenyl]acrylat beschichtet und dann mit linear polarisiertem Licht bestrahlt. Dabei wurde eine vorgegebene Struktur mittels einer Maske in die (PPN) Schicht photolithographisch eingeschrieben. Die neue Schicht (auf der PPN Schicht) wurde bei 90°C auf einer Wärmebank getrocknet, dann im Vakuumschank unter Vakuum bei 90°C mit Xenon-licht belichtet. Die eingeschriebene Originalstruktur blieb erhalten und wurde vom neuen Netzwerk getreu übernommen. Eine klare Doppelbrechung (Δn) war erkennbar. Diese Schicht fuhgiert als ein strukturierter optischer Retarder.

## Patentansprüche

1. Verbindungen der allgemeinen Formel 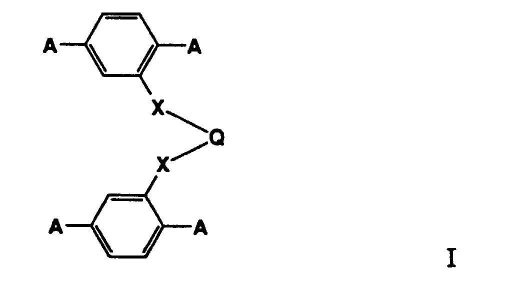 worin
X eine Einfachbindung, -COO-, -CH₂O-, -CONH- oder -C=N-;
Q n-Alkylen, c-Alkylen, 1,4-Phenylen, 4,4'-Biphenylen oder 2,6-Naphthylen bedeuten; und
A einen Rest der Formel 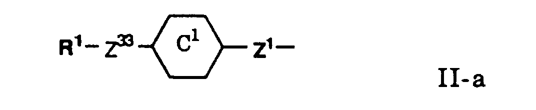 darstellt, worin
R¹ Acrylat, Methacrylat, 2-Chloracrylat, 2-Phenylacrylat, Vinyloxy oder Epoxy bedeutet;
Z³³ -(CH₂)_{m'}-, -(CH₂)_{m'}O-, -(CH₂)_{m'}COO- oder -(CH₂)_{m'}OOC- bedeutet;
m' eine ganze Zahl von 4 bis 12 ist;
Ring C¹ ein gegebenenfalls mit Halogen substituiertes 1,4-Phenylen, oder trans-1,4-Cyclohexylen bedeutet; und
Z¹ -CH₂CH₂-, -CH₂O-, -COO-, -OOC-, -(CH₂)₄- oder -(CH)₃O- bedeutet.

2. Verbindungen nach Anspruch 1 der Formeln 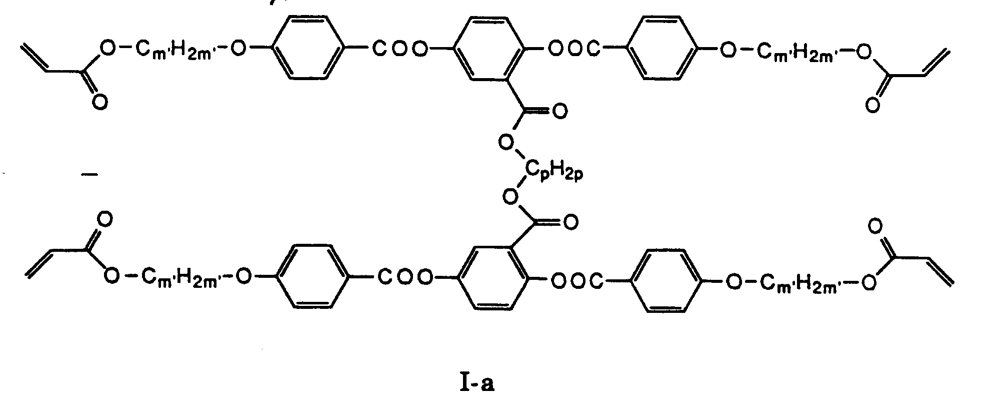 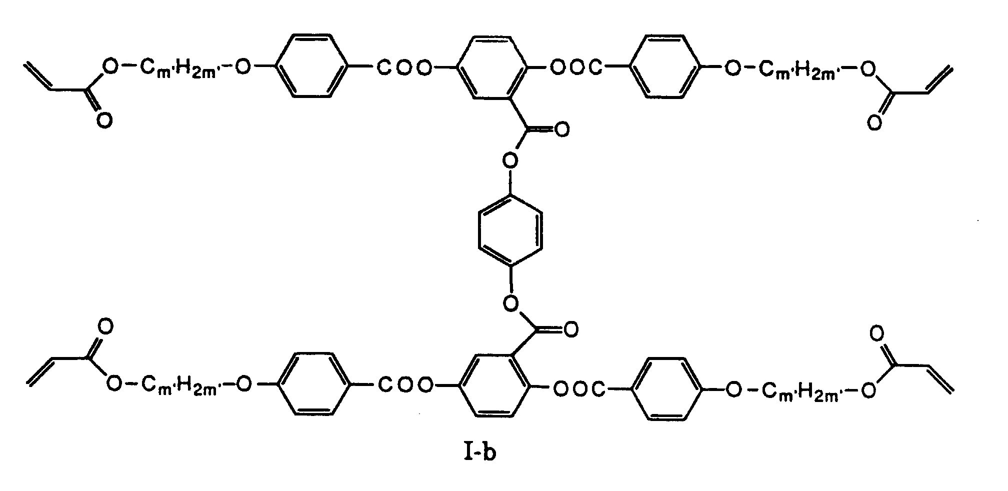 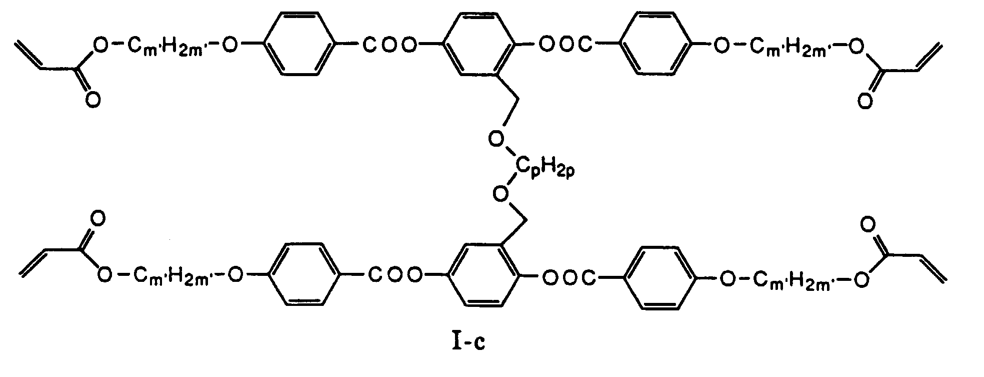 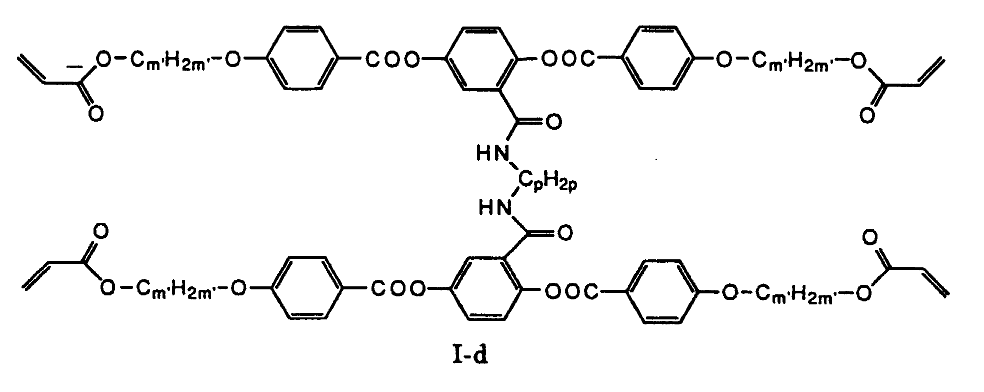 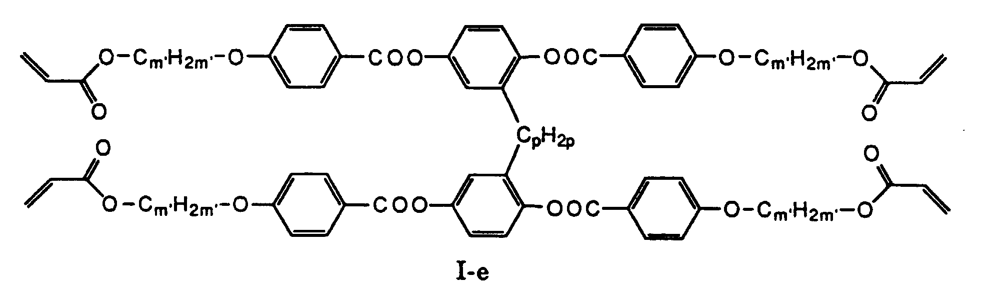 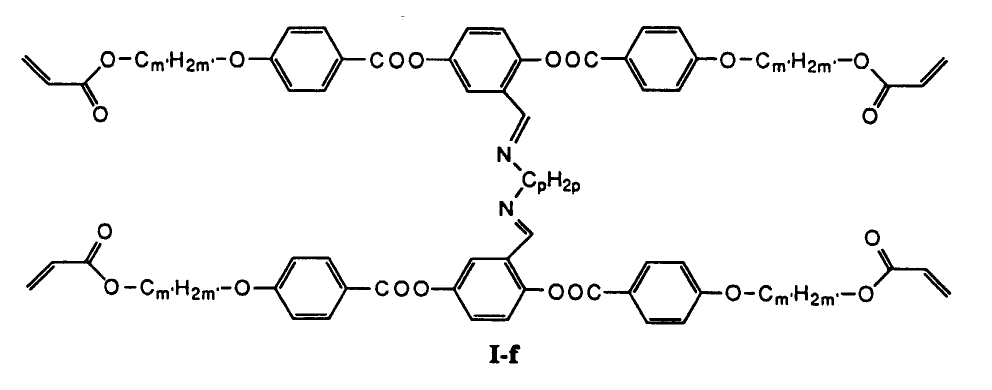 worin m' eine ganze Zahl von 4 bis 12 und p eine ganze Zahl von 2 bis 12 bedeutet.

3. Vernetzbare, flüssigkristalline Gemische bestehend aus mindestens 2 Komponenten, wovon mindestens eine Komponente eine Verbindung der in Anspruch 1 definierten Formel I ist.

4. Vernetzbare, flüssigkristalline Gemische gemäss Anspruch 3, dadurch gekennzeichnet, dass sie neben einer oder mehreren Verbindungen der Formel I, eine oder mehrere Verbindungen aus der Gruppe der Formeln 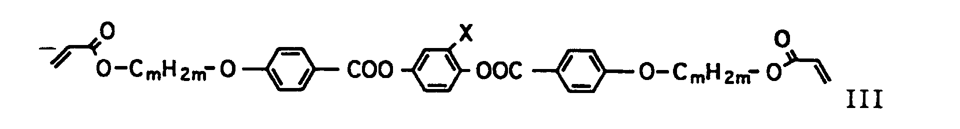 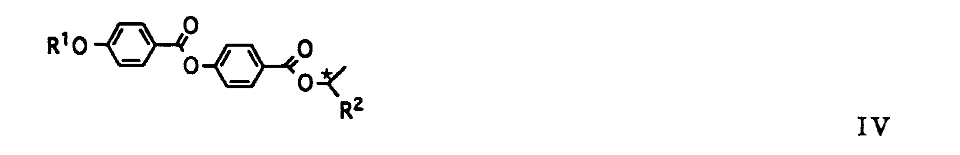 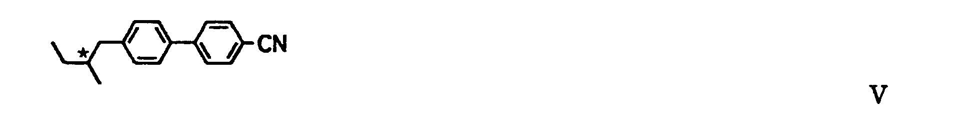 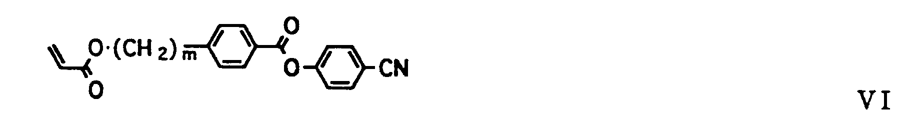 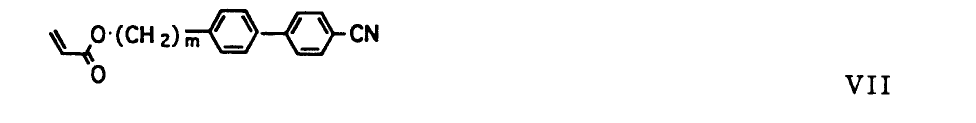 enthalten, worin R¹ und R² unabhängig voneinander Alkyl oder Alkenyl mit 2 bis 12 Kohlenstoffatomen darstellen; X Wasserstoff, Niederalkyl, Fluor, Brom, Chlor oder Cyano darstellt; und m eine ganze Zahl von 1 bis 16 bedeutet.

5. Verwendung von Verbindungen gemäss einem der Ansprüche 1 bis 2 in ihrem vernetzten Zustand für optische Bauelemente.

6. Verwendung von vernetzbaren flüssigkristallinen Gemischen gemäss einem der Ansprüche 3 oder 4 in ihrem vernetzten Zustand für optische Bauelemente.

## Claims

1. Compounds of the general formula 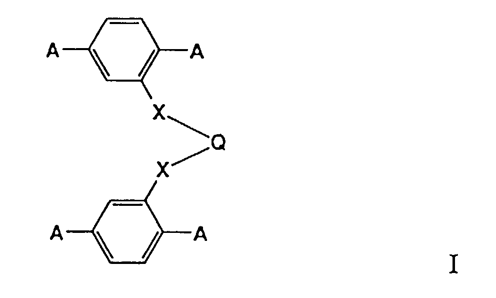 wherein
X signifies a single bond, -COO-, -CH₂O-, -CONH- or -C=N-;
Q represents n-alkylene, c-alkylene, 1,4-phenylene, 4,4'-biphenylene or 2,6-naphthylene; and
A represents a residue of the formula 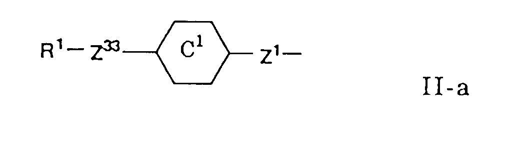
wherein
R¹ signifies acrylate, methacrylate, 2-chloroacrylate, 2-phenylacrylate, vinyloxy or epoxy;
Z³³ signifies -(CH₂)_{m'}-, -(CH₂)_{m'}O-, -(CH₂)_{m'}COO- or -(CH₂)_{m'}OOC-;
m' is a whole number of 4 to 12;
ring C signifies 1,4-phenylene, which is optionally substituted with halogen, or trans-1,4-cyclohexylene; and
Z¹ signifies -CH₂CH₂-, -CH₂O-, -COO-, -OOC-, -(CH₂)₄- or -(CH)₃O-.

2. Compounds according to claim 1, of the formulae 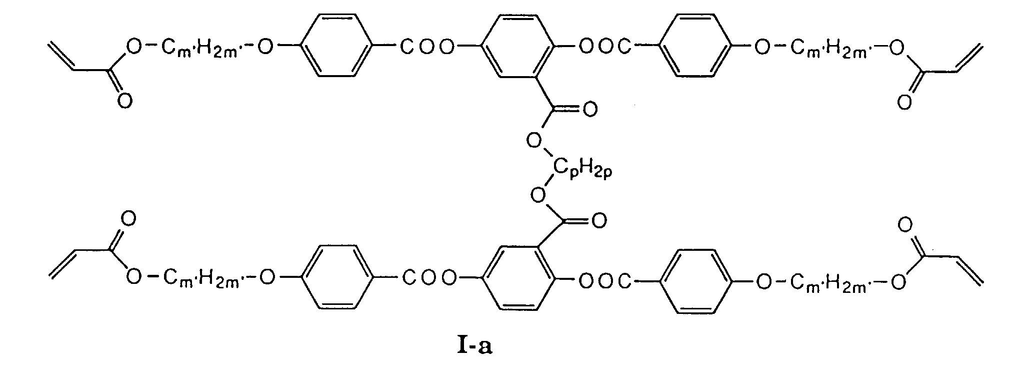 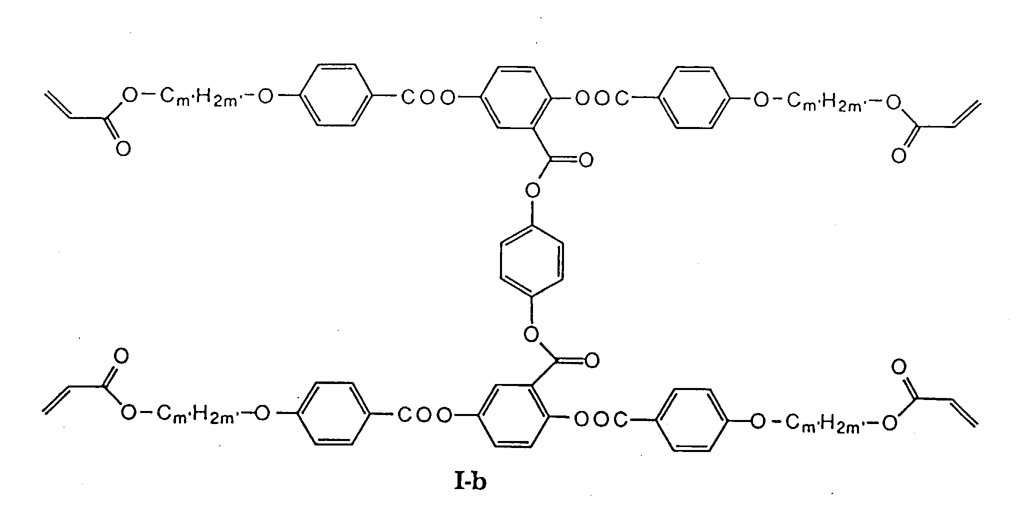 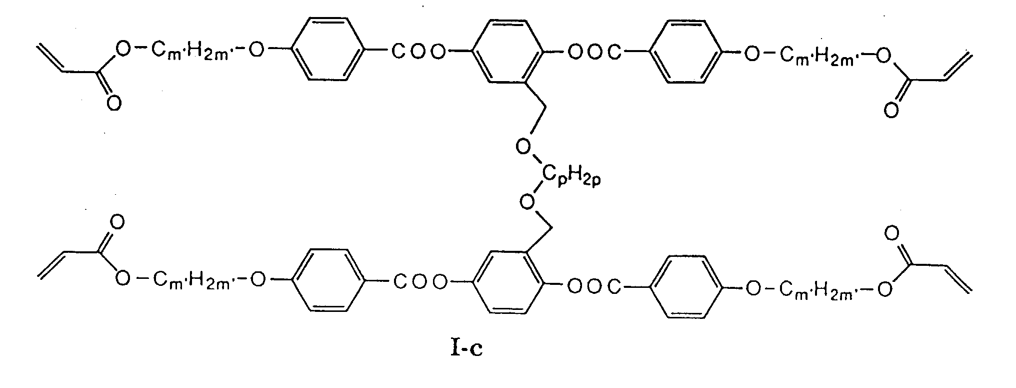 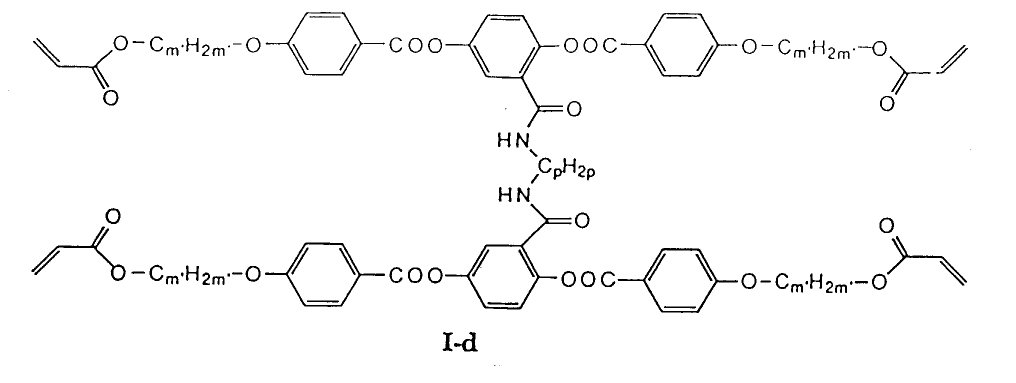 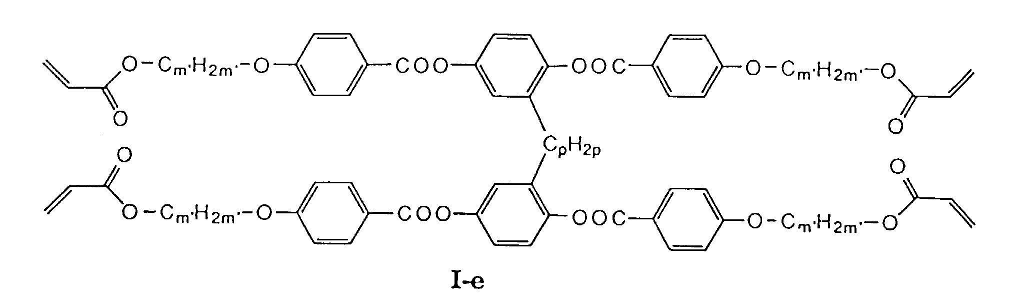 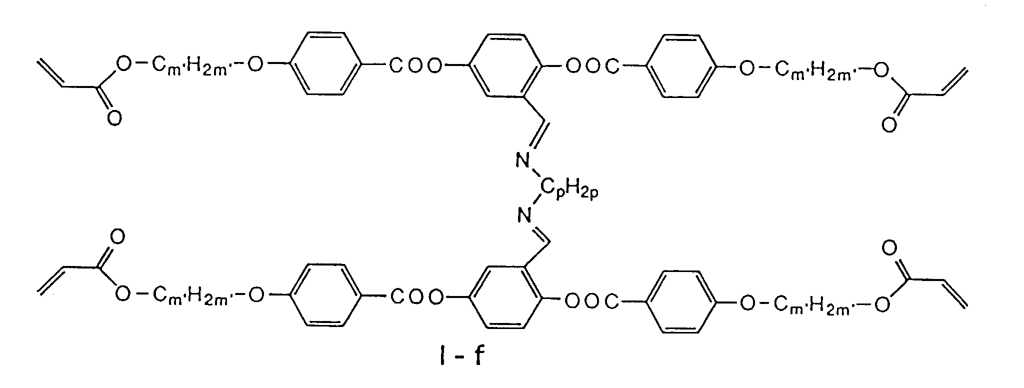 wherein m' signifies a whole number of 4 to 12 and p signifies a whole number of 2 to 12.

3. Cross-linkable, liquid crystalline mixtures consisting of at least 2 components, of which at least one component is a compound of formula I defined in claim 1.

4. Cross-linkable, liquid crystalline mixtures according to claim 3, characterized in that they contain, in addition to one or more compounds of formula I, one or more compounds from the group of the formulae 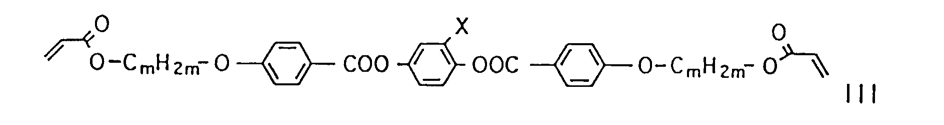 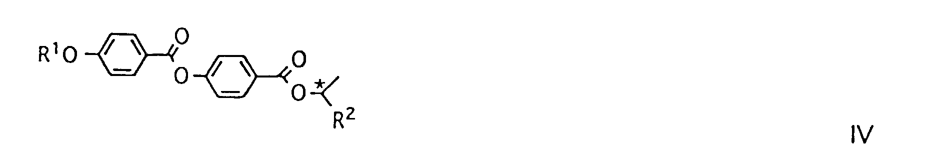 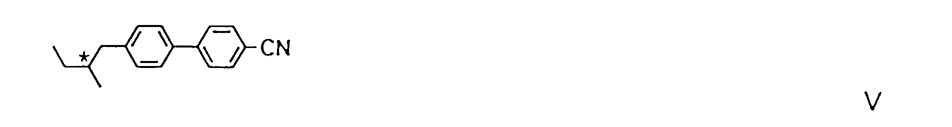 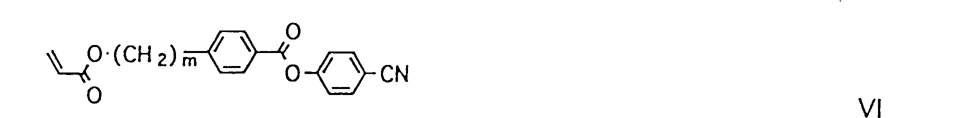 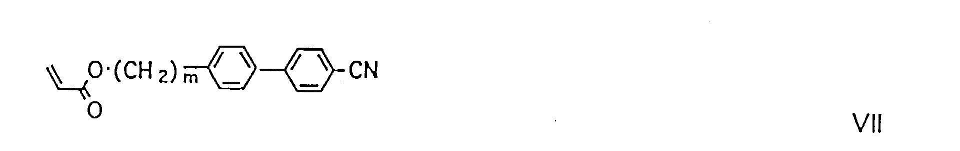 wherein R¹ and R² each independently represent alkyl or alkenyl with 2 to 12 carbon atoms; X represents hydrogen, lower alkyl, fluorine, bromine, chlorine or cyano; and m signifies a whole number of 1 to 16.

5. The use of compounds according to any one of claims 1 to 2 in their cross-linked state for optical building elements.

6. The use of cross-linkable liquid crystalline mixtures according to claim 3 or 4 in their cross-linked state for optical building elements.

## Revendications

1. Composés de formule générale 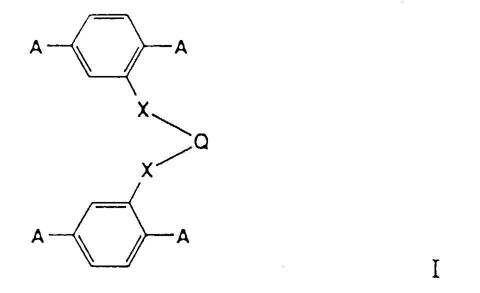 dans laquelle
X représente une simple liaison, un groupe -COO-, -CH₂O-, -CONH- ou -C=N-;
Q représente un groupe n-alkylène, c-alkylène, 1,4-phénylène, 4,4'-biphénylène ou 2,6-naphtylène; et
A représente un groupe de formule 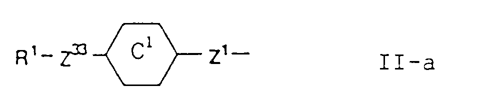
dans laquelle
R¹ représente un groupe acrylate, méthacrylate, 2-chloroacrylate, 2-phénylacrylate, vinyloxy ou époxy;
Z³³ représente un groupe -(CH₂)_{m'}-, -(CH₂)_{m'}O-, -(CH₂)_{m'}COO- ou -(CH₂)_{m'}OOC-;
m' est un nombre entier de 4 à 12;
le cycle C¹ représente un groupe 1,4-phénylène, ou trans-1,4-cyclohexylène éventuellement substitué avec des atomes d'halogène; et
Z¹ représente un groupe -CH₂CH₂-, -CH₂O-, -COO-, -OOC-, -(CH₂)₄- ou -(CH)₃O-.

2. Composés selon la revendication 1 de formules 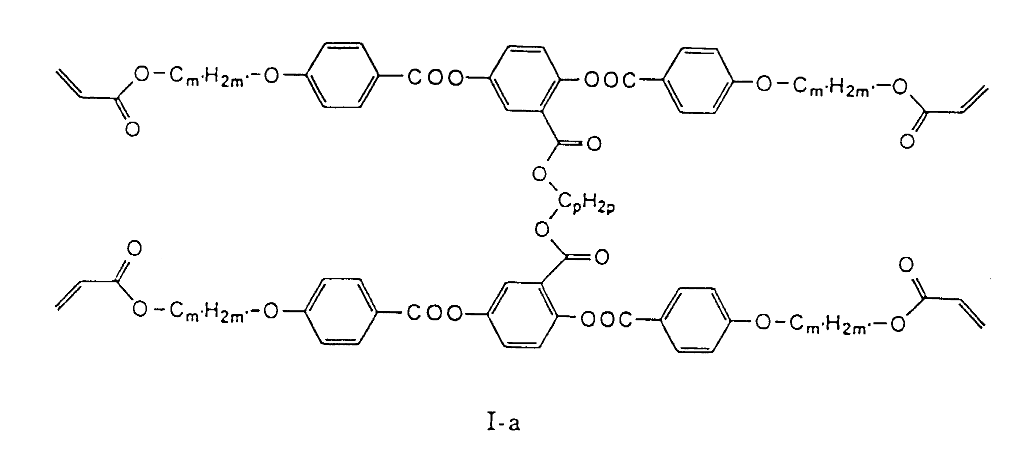 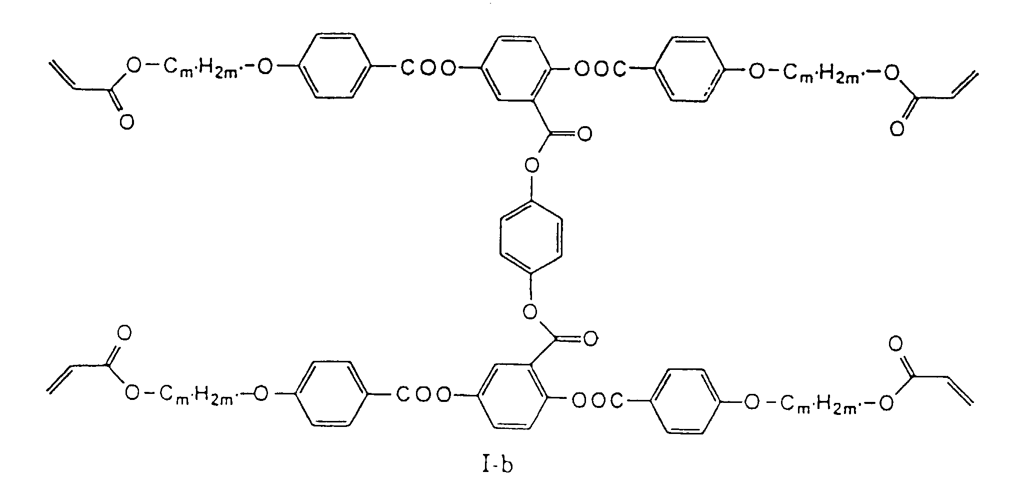 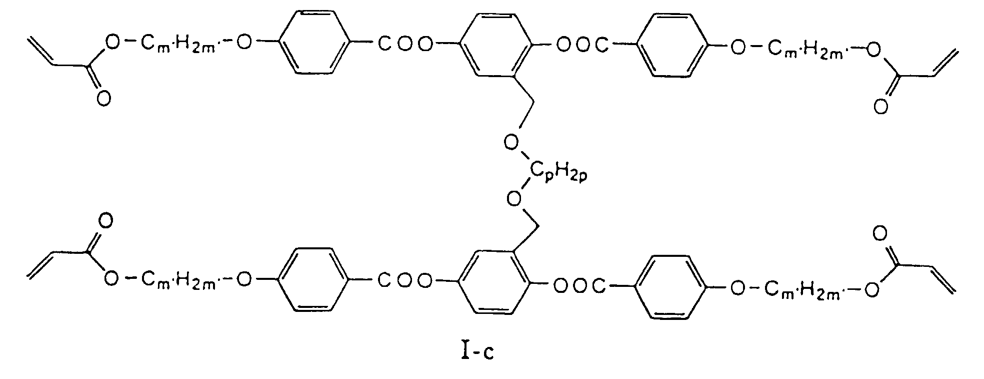 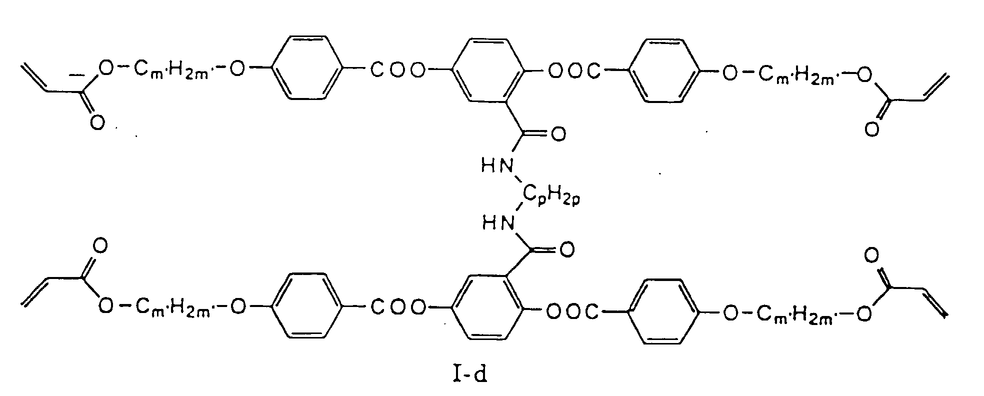 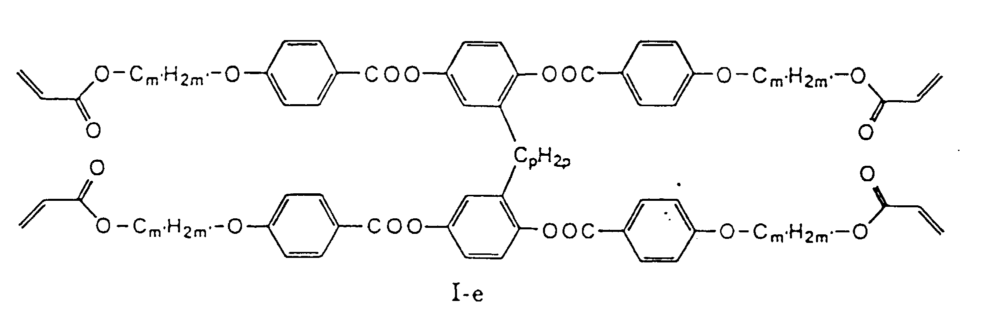 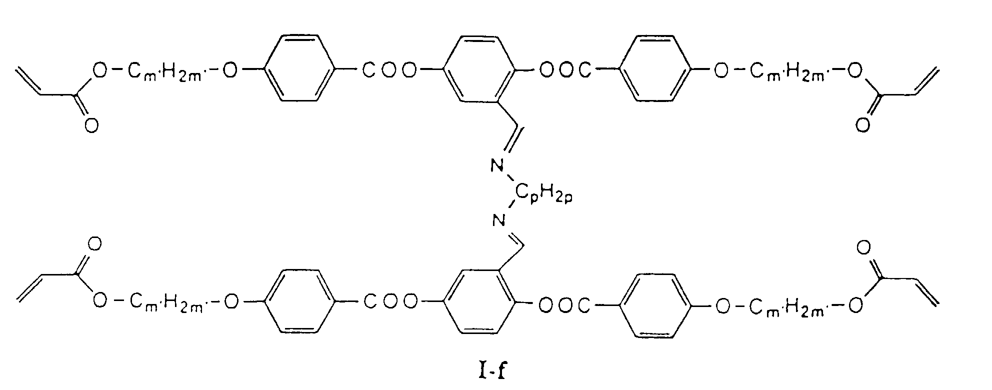 dans lesquelles m' représente un nombre entier de 4 à 12 et p un nombre entier de 2 à 12.

3. Mélanges de cristaux liquides, réticulables, constitués d'au moins 2 composants, parmi lesquels au moins un composant est un composé de formule I définie à la revendication 1.

4. Mélanges de cristaux liquides selon la revendication 3, réticulables, caractérisés en ce qu'ils contiennent en plus d'un ou de plusieurs composés de formule I, un ou plusieurs composés choisis parmi les formules 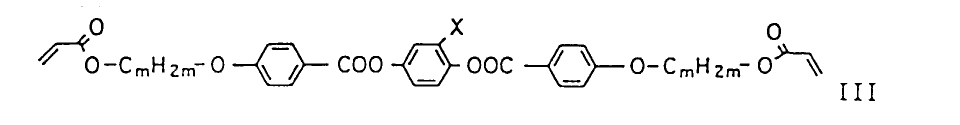 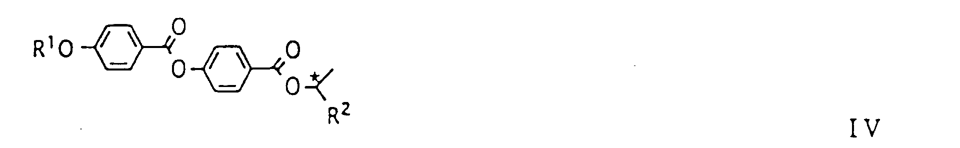 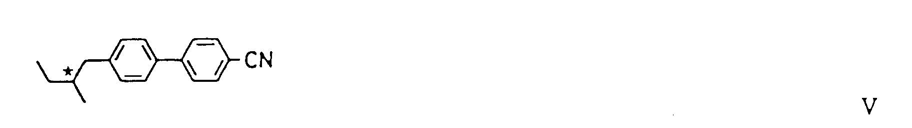 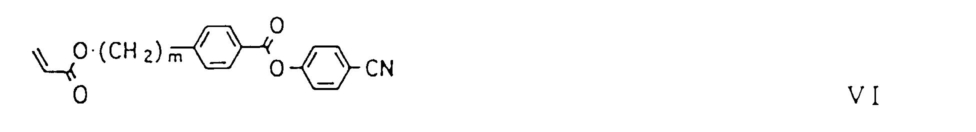 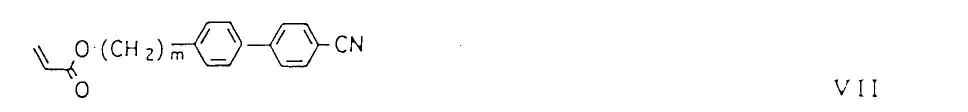 dans lesquelles R¹ et R² représentent indépendamment l'un de l'autre un groupe alkyle ou alcényle ayant 2 à 12 atomes de carbone; X représente un atome d'hydrogène, un groupe alkyle inférieur, un atome de fluor, de brome, de chlore ou un groupe cyano; et m représente un nombre entier de 1 à 16.

5. Utilisation de composés selon l'une des revendications 1 à 2 dans leur état réticulé pour des composants optiques.

6. Utilisation de mélanges de cristaux liquides réticulables selon l'une des revendications 3 ou 4 dans leur état réticulé pour des composants optiques.
